(19) Europäisches Patentamt
European Patent Office
Office européen des brevets

(11) EP 4 772 519 A1

(12) **EUROPEAN PATENT APPLICATION**
published in accordance with Art. 153(4) EPC

(43) Date of publication:
**08.07.2026 Bulletin 2026/28**

(21) Application number: **24890359.3**

(22) Date of filing: **29.09.2024**

(51) International Patent Classification (IPC):
***C07H 15/08** (2006.01)* ***C08F 212/32** (2006.01)*
***C08F 220/56** (2006.01)* ***C08F 220/58** (2006.01)*
***C08F 226/10** (2006.01)* ***C09K 8/588** (2006.01)*

(52) Cooperative Patent Classification (CPC):
**C07H 15/08; C08F 212/32; C08F 220/56; C08F 220/58; C08F 226/10; C09K 8/588**

(86) International application number:
**PCT/CN2024/122296**

(87) International publication number:
**WO 2025/103001 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.11.2023 CN 202311522670**
**15.11.2023 CN 202311522771**

(71) Applicants:
- **China Petroleum & Chemical Corporation**
  **Beijing 100728 (CN)**
- **Sinopec (Beijing) Research Institute of Chemical Industry Co., Ltd.**
  **Beijing 100013 (CN)**

(72) Inventors:
- **LIU, Xi**
  **Beijing 100013 (CN)**
- **YI, Zhuo**
  **Beijing 100013 (CN)**
- **HU, Xiaona**
  **Beijing 100013 (CN)**
- **LI, Yajing**
  **Beijing 100013 (CN)**
- **ZHANG, Ruiqi**
  **Beijing 100013 (CN)**
- **YANG, Jinbiao**
  **Beijing 100013 (CN)**
- **FAN, Rong**
  **Beijing 100013 (CN)**
- **SHANG, Dansen**
  **Beijing 100013 (CN)**
- **ZHAO, Ruotong**
  **Beijing 100013 (CN)**
- **WU, Jialei**
  **Beijing 100013 (CN)**

(74) Representative: **Hoffmann Eitle**
**Patent- und Rechtsanwälte PartmbB**
**Arabellastraße 30**
**81925 München (DE)**

# (54) GLYCOSIDE GROUP-CONTAINING COMPOUND AND USE THEREOF

(57) A glycoside group-containing compound and a preparation method therefor and a use thereof. The glycoside group-containing compound has a structure represented by formula (1), and has a high emulsifying capability. The polymer obtained by polymerization of the glycoside group-containing compound can improve the water-flooding recovery rate of a conventional oil reservoir and a common heavy oil reservoir.

EP 4 772 519 A1

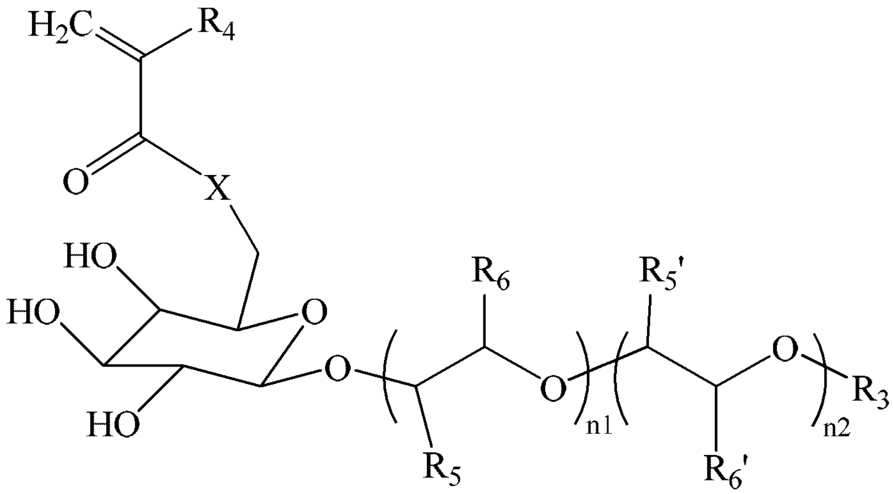

(1)

## Description

### CROSS-REFERENCE TO RELATED APPLICATIONS

The application claims the benefit of the China patent application No. "202311522670.5", filed on November 15, 2023, and the China patent application No. "202311522771.2", filed on November 15, 2023, the contents of which are specifically and entirely incorporated herein by reference.

### TECHNICAL FIELD

The present invention relates to the technical field of preparing a new compound, concretely to a glycoside group-containing compound and a preparation method therefor and a use thereof, particularly to the development and use of a new compound suitable for the oil recovery field.

### BACKGROUND ART

Heavyoil occupies about 70% share of the explored petroleum resources in the world. However, heavy oil has the defects of high viscosity and low driving efficiency, thermal recovery is the main development mode, and there are problems that a large amount of remaining oil cannot be extracted from wells in later period, and the energy consumption is high. Cold oil recovery, which generally improves fluidity of heavy oil with chemical methods, has the advantages of simple production process and low costs, thus it is an important development tendency.

The addition of small molecule water-soluble viscosity reducers can improve recovery ratio by reducing viscosity, however, the cold production heavy oil reservoirs generally have strong heterogeneity, the small molecule viscosity reducer solution has the defects such as low viscosity, severe fingering effect and unbalanced oil displacement. When the viscosity reducers are used in compounding with polymers, the oil displacement effect can be improved, but chromatographic separation exists, and deep profile-control flooding and oil washing cannot be achieved. In addition, the driving force is weak in the deep reservoirs, but the usual operation of emulsifying heavy oil with a viscosity reducer needs to be performed under the strong stirring action. Therefore, it is difficult to emulsify heavy oil under the oil reservoir seepage power.

Because the heavy oil has the high contents of asphaltene and colloid, high viscosity, the heavy oil cannot be easily extracted. Therefore, it is urgent to develop a functional polymer which can affect the thick oil to replace the weak hydrogen bonding between the thick oil, and has the ability of controlling the fluidity and interfacial activity, and can emulsify the heavy oil under the low reservoir driving force, the development of functional polymer has an important significance for improving the recovery rate of heavy oil by cold production.

### SUMMARY OF THE INVENTION

The present invention aims to overcome the problems in the prior art and provide a glycoside group-containing compound and a use thereof.

In order to achieve the above object, the present invention provides a glycoside group-containing compound having a structure represented by formula (1).

The present invention also provides a glycoside group-containing polymer, the polymer comprises a structural unit C represented by formula (C) and a structural unit D represented by formula (D).

The present invention further provides a method for preparing a glycoside group-containing polymer and the polymer produced with said method.

The present invention also provides a method for reducing viscosity of heavy oil, the method comprises: contacting a solution of the polymer with said heavy oil.

Furthermore, the present invention provides a use of the compound and the polymer in the viscosity reduction of heavy oils.

The compounds of the present invention have strong emulsifying capability and a longer emulsification time in heavy oil emulsification tests. The polymer obtained by copolymerizing the compounds of the present invention with other monomers has a strong salt resistance, and exhibits a water phase thickening function, can improve oil-water fluidity ratio during the oil displacement process, and adjust heterogeneity of oil reservoirs. In particular, the compounds have a high viscosity reduction rate for the heavy oil having a viscosity lower than or equal to 3,000mPa·s at the oil reservoir temperature under the low driving force environment of oil reservoir with a mineralization degree lower than or equal to 50,000 mg/L and a temperature lower than or equal to 70°C, can improve the water-flooding recovery rate of a conventional oil reservoir and a common heavy oil reservoir.

In particular, compared to alkyl groups (the sum of n1 and n2 is zero), the introduction of alcohol ether groups (the

sum of n1 and n2 is not zero) allows the structural unit provided by a glycoside group-containing compound to significantly improve the above properties of the polymer at a low content. Still further, the introduction of two specific alcohol ether groups can significantly improve the above properties of the polymer at a circumstance of using a lower content of the structural unit provided by the compound.

## DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

The first aspect of the present invention provides a glycoside group-containing compound (or modified glycoside), the compound has a structure represented by formula (1):

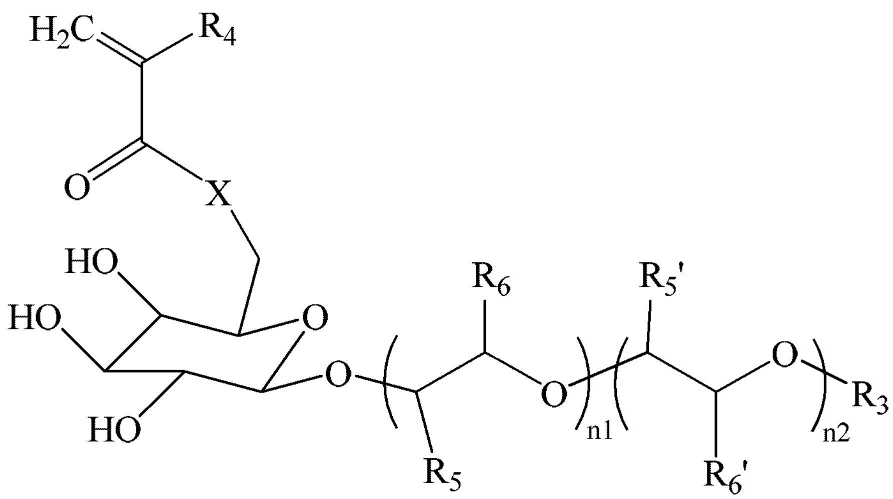

Formula (1)

In formula (1), $R_3$ is C4-C20 (e.g., C5, C6, C8, C10, C12, C14, C16, C18, C20) alkyl or C7-C20 alkylphenyl (propylphenyl, butylphenyl, pentylphenyl, heptylphenyl, octylphenyl, nonylphenyl, decylphenyl, dodecylphenyl), $R_4$, $R_5$, $R_6$, $R_5$' and $R_6$' are each independently H or C1-C4 alkyl, X is O or NQ, Q is H or C1-C4 alkyl, the sum of n1 and n2 is a natural number within the range of 0-20.

In the invention, when X is O, the sum of n1 and n2 is not equal to 0, i.e., when X is O, the sum of n1 and n2 is a natural number ranging from 1 to 20 (n1+n2=1-20), and when X is NQ (e.g., imino, NH), the sum of n1 and n2 is a natural number ranging from 0 to 20 (n1+n2=0-20).

Preferably, $R_3$ is C6-C18 n-alkyl, C6-C18 iso-alkyl or C7-C18 alkylphenyl, more preferably n-hexyl, n-dodecyl, n-decyl, n-octyl, n-tetradecyl, 2-ethylhexyl or 11-methyldodecyl.

Preferably, $R_4$, $R_5$, $R_6$, $R_5$' and $R_6$' are each independently H or methyl.

Preferably, $R_5$' and $R_6$' are each independently H or methyl and $R_5$' and $R_6$' are different, both $R_5$ and $R_6$ are H.

More preferably, $R_5$' is methyl.

Preferably, $R_5$ and $R_6$ are each independently H or methyl, more preferably H.

More preferably, $R_5$' is methyl, $R_6$' is H, both $R_5$ and $R_6$ are H. The compounds of the preferred embodiments have more excellent properties.

Preferably, X is O or an imino group (-NH-).

Preferably, when X is O, n1 is 1, 2, 3, 4, 5, 6, 7, 8, 9 or 10, n2 is 1, 2, 3, 4, 5, 6, 7, 8, 9, 10, 11, 12, 13, 14, 15, 16, 17, 18, 19 or 20.

Preferably, when X is NQ (e.g., -NH-), n1 is 0, 1, 2, 3, 4, 5, 6, 7, 8, 9 or 10, n2 is 0, 1, 2, 3, 4, 5, 6, 7, 8, 9, 10, 11, 12, 13, 14, 15, 16, 17, 18, 19 or 20.

According to a preferred embodiment of the invention, n2/n1=1-10, more preferably, n2/n 1=1.5-5.5.

According to a preferred embodiment of the invention, the compound has a structure represented by formula (1-1) or formula (1-2):

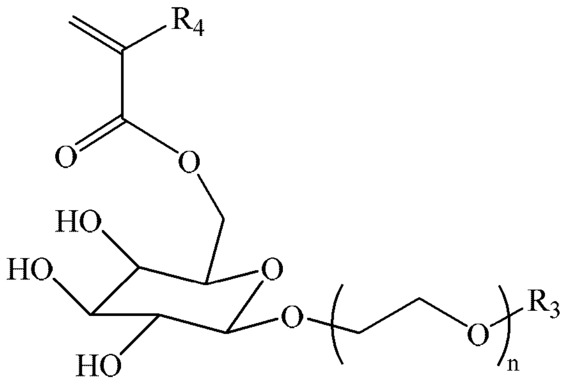

Formula (1-1)

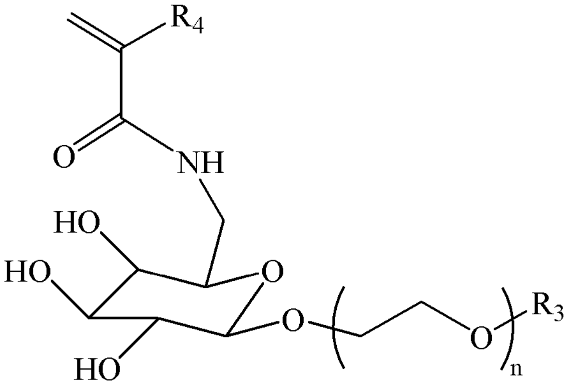

Formula (1-2)

Wherein n is a natural number ranging from 0 to 20 (preferably 0, 1, 2, 3, 4, 5, 6, 7, 8, 9 or 10); $R_3$ and $R_4$ are as previously mentioned.

The compounds of the present invention have strong emulsifying capability, exhibit excellent interfacial activity, when the compound is adsorbed onto the oil-water interface, it can reduce the oil-water interfacial tension, thereby contributing to the formation of a surface tension gradient.

The present invention has no particular requirement on the specific preparation method for preparing the compound described in the aforementioned first aspect, and those skilled in the art can determine a suitable synthesis method in accordance with the structural formula provided by the present invention in combination with the known knowledge in the organic synthesis field, or those skilled in the art can also prepare the aforementioned compound by using the specific example (substitute raw material) provided later in the present invention. However, in order to further increase yield of the compound, the second aspect of the present invention provides a method for preparing the compound, the method comprises: mixing at least one of the glycoside compounds represented by formula (2) with at least one of the double bond-containing compounds represented by the formula (3) and carrying out the reaction:

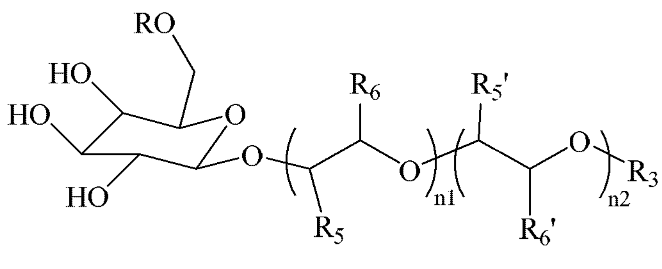

Formula(2)

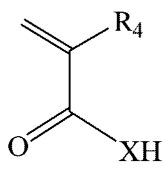

Formula (3)

Wherein R is selected from H, trifluoromethylsulfonyl or p-toluenesulfonyl; X, $R_3$, $R_4$, $R_5$, $R_6$, $R_5$', $R_6$', n1 and n2 are as previously described herein, and will not be further elaborated.

In the invention, the natural fatty alcohol raw material is mostly present in a form of mixed alcohol (e.g., C8 coexists with C10, C12 coexists with C14). Therefore, when a glycoside compound represented by formula (2) is prepared by using the natural fatty alcohol raw material, the resultant product is also a mixed glycoside, which has substantially the same properties as the single glycoside and can react with the double bond-containing compound represented by formula (3), and the resultant product is also a mixture thereof. It is clear that such a mixture also falls into the protection scope of the

present invention.

In the present invention, the dosages of the raw materials in the preparation process of said compound are not specifically limited. In some embodiments of the invention, the glycoside compound is used in an amount of 0.65-1.5mol relative to 1mol of the double bond-containing compound.

In some embodiments of the invention, the reaction temperature may be within the range of 0-200°C (such as below room temperature (0-25°C), or 80-100°C), preferably within the range of 20-150°C. The reaction time may be 5-12 hours, preferably 6-10 hours.

In some embodiments of the invention, the mixing is carried out in the presence of an activator, which may be selected from diisopropyl azodicarboxylate and/or triphenylphosphine, preferably having a molar ratio of diisopropyl azodicarboxylate and triphenylphosphine within the range of 0.5-1.5, the activator serves to activate the hydroxyl group and makes it easily removable. The dosage of said activator is preferably 2-5mol relative to 1mol of the double bond-containing compound. The use of said activator is conducive to lowering the esterification reaction temperature (it can be controlled to room temperature). When an activator is used, in order to avoid oxidation in air, the esterification reaction is carried out in an inert atmosphere, which may be provided by nitrogen gas and/or an inert gas. In the context of the present invention, inert gas refers to the simple substance gas corresponding to all the elements of Group 0 of the Periodic Table of Elements, including helium (He), neon (Ne), argon (Ar), krypton (Kr), xenon (Xe), radon (Rn), etc.

In some embodiments of the invention, the mixing is carried out in the presence of triflic acid, the primary function of which is to activate the R group to make it easier to remove. The triflic acid is preferably used in an amount of 0.1-0.3mol relative to 1mol of the double bond-containing compound. The use of triflic acid advantageously accelerates the reaction rate, expedites the forward progress of the reaction. When triflic acid is used, to avoid hydrolysis thereof, the reaction is carried out in an inert atmosphere, which may be provided by nitrogen gas and/or an inert gas.

In some embodiments of the invention, the method may further comprise purifying after the reaction is complete. The purification method is not particularly limited in the invention, for example, purification may be performed using extractive spin drying, recrystallization, column chromatography and the like. According to a specific embodiment of the present invention, the purification comprises: cooling the reaction system, performing the reduced pressure distillation to remove the solvent and unreacted double bond-containing compound. In another specific embodiment of the invention, the purification may further comprise: subjecting the reaction system to the reduced pressure distillation to remove the solvent, and removing the unreacted raw materials through a chromatography column separation.

In the present invention, when X is O, in accordance with a preferred embodiment of the invention, the process comprises: contacting at least one of double bond-containing compounds (acrylic acid-based compounds) represented by formula (3a) with at least one glycoside compound represented by formula (2a) under the esterification conditions to obtain a modified glycoside product, wherein n is as previously described.

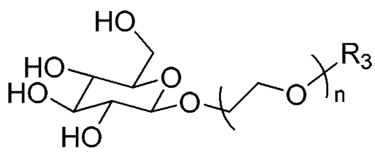

Formula (2a)

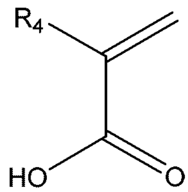

Formula (3a)

The contacting of the double bond-containing compound represented by formula (3a) with the glycoside compound represented by formula (2a) may be performed in an organic solvent, which may be various solvents capable of dissolving the reaction substrate, such as C6-C10 benzene series and/or N,N-dialkyl formamide. The alkyl group in the N,N-dialkyl formamide is preferably C1-C3 alkyl (e.g., methyl, ethyl, propyl, or isopropyl). Preferably, the organic solvent is at least one selected from the group consisting of toluene, xylene, and N,N-dimethyl formamide. The dosage of said organic solvent is not particularly limited, as long as it is sufficient to mainly dissolve the glycoside compound and the double bond-containing compound. The mode of contacting the double bond-containing compound represented by formula (3a) with the glycoside compound represented by formula (2a) is not particularly defined, the contacting may be performed in the form of a solution. In some embodiments of the present invention, the preparing steps comprise: adding the glycoside compound dissolved in an organic solvent to the double bond-containing compound solution, and then placing the materials under the esterification reaction conditions. The esterification reaction of the double bond-containing compound represented by formula (3a) with the glycoside compound represented by formula (2a) may be performed in the presence of an activating agent, which is as previously described. It is understandable that replacing the compound represented by formula (2a) with the compound represented by formula (2d) and contacting it with the double bond-containing compound represented by formula (3a) (repeating the above steps), the glycoside group-containing compounds with more other structures can be obtained.

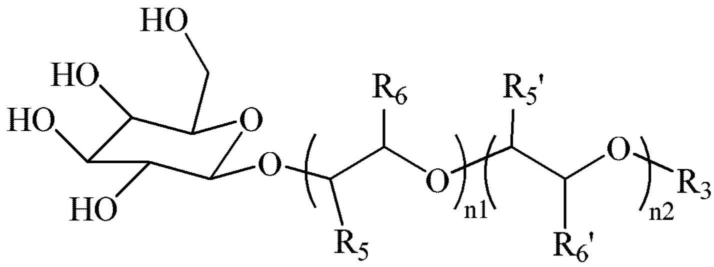
Formula (2d)

In the present invention, when X is NH, according to another preferred embodiment of the invention, the method comprises: mixing at least one of the glycoside compounds represented by formula (2b) with at least one of the double bond-containing compounds represented by formula (3b) and carrying out the reaction under the amidation reaction conditions, wherein n is as previously described.

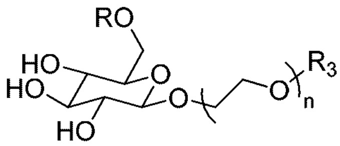
Formula (2b)
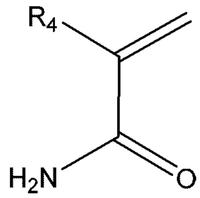
Formula (3b)

The amidation reaction may be performed at a temperature of 10-200°C (such as below room temperature (0-25°C) or 80-100°C). The amidation reaction may be performed for 5-12h, preferably 6-10h. When R is H, the amidation reaction is preferably performed under the condition of below room temperature (0-25°C). In the invention, in order to conveniently separate the target product and further improve the yield of said target product, according to a preferred embodiment of the present invention, the method further comprises contacting at least one of the glycoside compounds represented by formula (2a) with a sulfonating agent under the esterification reaction conditions to obtain the glycoside sulfonate ester represented by formula (2c), then carrying out the reaction (amidation reaction) on the glycoside sulfonate ester represented by formula (2c) and the double bond-containing compound represented by formula (3b). The sulfonating agent is p-toluenesulfonyl halide and/or triflic anhydride. The use of triflic anhydride as a sulfonating agent makes the product separation to be easier. The compound of the present invention is prepared through such a two-step reaction, only the reduced pressure distillation is required to obtain the intermediate or the target product, the separation process has a higher simplicity, efficiency and practicability.

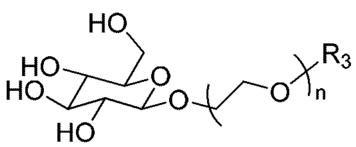
Formula (2a)
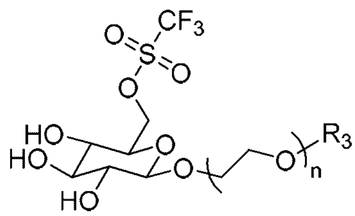
Formula (2c).

In some embodiments of the present invention, the sulfonating agent is used in an amount of 0.8-1.5mol relative to 1mol of the glycoside compound.

In some embodiments of the present invention, the conditions for contacting the glycoside compound represented by formula (2a) with the sulfonating agent to carry out the esterification reaction comprise: a reaction time of 5-12h, preferably 6-10h. The temperature for contacting the glycoside compound represented by formula (2a) with the sulfonating agent to carry out the esterification reaction may be below room temperature (e.g., 0-25°C).

In some embodiments of the invention, an organic base is also introduced into the esterification reaction system consisting of the glycoside compound represented by formula (2a) and the sulfonating agent, i.e., the esterification reaction is carried out in the presence of an organic base. The organic base is capable of providing a reaction medium for the esterification reaction and neutralizing the acid (e.g., hydrochloric acid) produced during the reaction process. Preferably, the organic base is used in an amount of 10-50mol relative to 1mol of the glycoside compound. The organic base is preferably at least one of the C4-C10 nitrogen-containing heterocyclic compounds, more preferably, the organic base is at least one selected from the group consisting of pyridine, triethylamine and piperidine. The esterification reaction of the glycoside compound represented by formula (2a) and the sulfonating agent may be carried out in the presence of an

activator, which is as previously described.

In some embodiments of the invention, the conditions for mixing the glycoside sulfonate ester represented by formula (2c) with the double bond-containing compound comprise: a temperature of 10-200°C, preferably 80-100°C, and a time of 5-12h, preferably 6-10h.

In some embodiments of the invention, in order to expedite the reaction progress, the mixing of the glycoside sulfonate ester represented by formula (2c) and the double bond-containing compound is conducted in the presence of sodium hydride. The sodium hydride may be used in an amount of 1-3mol relative to 1mol of the double bond-containing compound.

In some embodiments of the present invention, the mixing of the glycoside sulfonate ester represented by the formula (2c) and the double bond-containing compound is performed in an organic solvent, preferably, the organic solvent is at least one selected from the group consisting of tetrahydrofuran, glyme and dioxane. The dosage of said organic solvent is not particularly limited, as long as it mainly dissolves the glycoside sulfonate ester and the double bond-containing compound.

It is understandable that replacing the compound represented by formula (2b) with the compound represented by formula (2e) and contacting it with the double bond-containing compound represented by formula (3b) (repeating the above steps), the glycoside group-containing compounds with more other structures can be obtained.

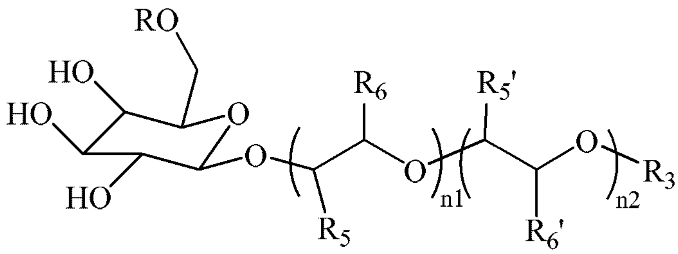

Formula (2e)

The third aspect of the present invention provides a glycoside group-containing polymer, is characterized in that the polymer comprises a structural unit C represented by formula (C) (preferably formula (C')):

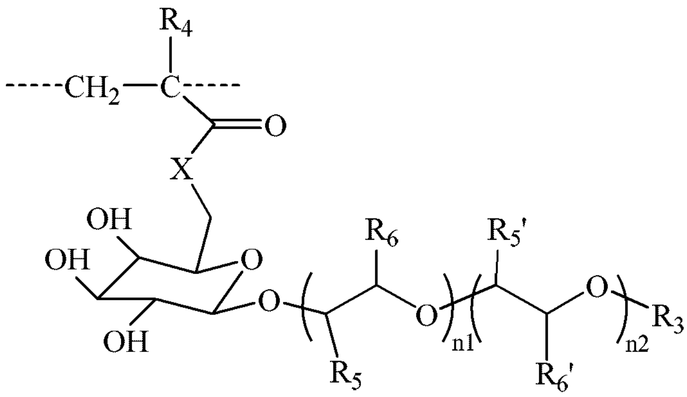

Formula (C)

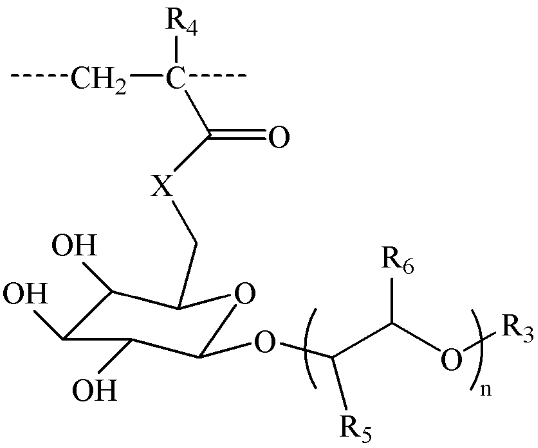

Formula (C')

$R_3$, $R_4$, $R_5$, $R_6$, $R_5$', $R_6$', n1 and n2 and n are as previously described, but the circumstance that n=n1=n2=0 is also included in the polymer range of the present invention, i.e. n1 + n2 in formula (C) or n in formula (C) is a natural number of 0-20, such as 0, 1, 2, 3, 4, 5, 6, 7, 8, 9, 10, 11, 12, 13, 14, 15, 16, 17, 18, 19 or 20.

According to the invention, the content of said structural unit C is preferably 0.5wt% or more of the total weight of polymer, more preferably 1-10wt% of the total weight of polymer. According to a preferred embodiment of the present invention, when the polymer comprises the structural unit C represented by formula (C'), the content of said structural unit C is 3wt% or more. According to another preferred embodiment of the present invention, the polymer comprises the structural unit C represented by formula (C), the part

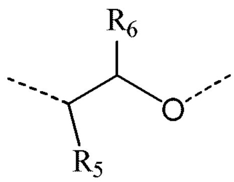

and part

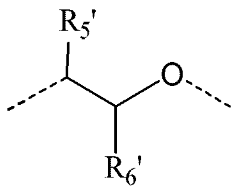

in the formula (C) are present simultaneously but different (correspondingly, the two parts of formula (1) are present simultaneously but are different), the content of the structural unit C is preferably lower than 3wt%, more preferably 1-2.5wt%. The introduction of two specific alcohol ether groups can significantly improve the polymer performance with the lower content of the structural unit provided by the compound.

According to the present invention, the polymer may also comprise structural units represented by formula (C-1) and/or formula (C-2):

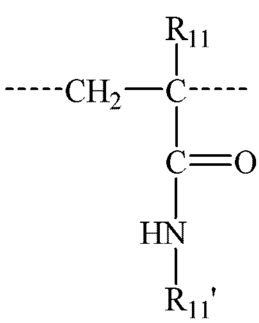

Formula (C-1)

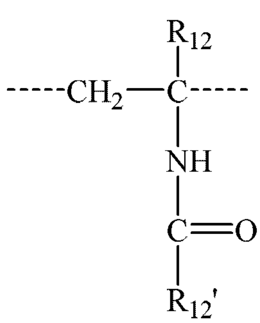

Formula (C-2)

Wherein $R_{11}$ and $R_{12}$ are each independently H or C1-C4 alkyl (preferably methyl); $R_{11}$' and $R_{12}$' are each independently H, C1-C4 alkyl (preferably methyl), $SO_3M$ substituted C2-C6 alkyl (preferably $-C(CH_3)_2CH_2SO_3M$), P(O)$(OM)_2$ substituted C2-C6 alkyl (preferably $-C(CH_3)_2CH_2P(O)(OM)_2$), M is H or an alkali metal element. The content of said structural unit represented by formula (C-1) may be more than 55wt%, preferably 65-97wt% of the total weight of polymer (or the weight ratio of said structural unit represented by formula (C-1) to the structural unit C is (1-1.8): (0.005-0.3), preferably (1-1.7): (0.006-0.15) ). More preferably, the content of said structural unit represented by formula (C-2) may be 1-10wt%, preferably 1.5-5wt% of the total weight of polymer. Preferably, the structural unit represented by formula (C-1) is selected from the group consisting of the structural unit A represented by formula (A) and/or the structural unit represented by formula (B-2). Preferably, the structural unit represented by formula (C-2) is selected from the structural unit D represented by formula (D).

More preferably, the weight ratio of said structural unit C to said structural unit D is (5-300): (5-300), further preferably (6-150): (8-200), most preferably 0.5-10 (e.g., 0.5, 1, 1.5, 2, 2.5, 3, 4, 5, 5.5, 6, 7, 8, 9 or 10).

According to a preferred embodiment of the present invention, the polymer comprises a structural unit A represented by formula (A), a structural unit B represented by formula (B-1) or (B-2), a structural unit C represented by formula (C) and a structural unit D represented by formula (D);

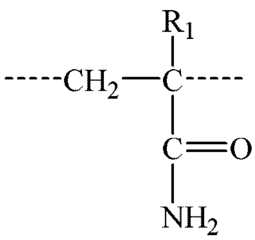 Formula (A) 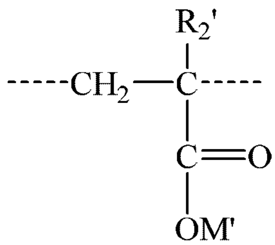 Formula (B-1),

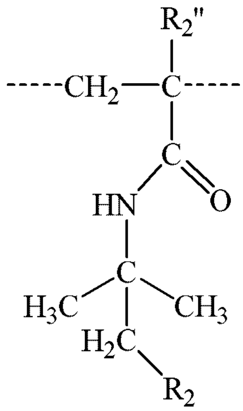 Formula (B-2) 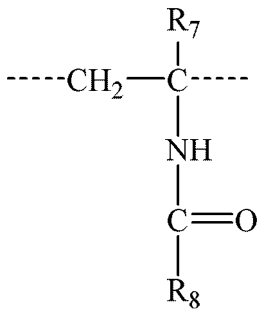 Formula (D);

Wherein $R_1$, $R_2$', $R_2$", $R_7$ and $R_8$ are each independently H or C1-C4 alkyl, $R_2$ is $SO_3M$ or $P(O)(OM)_2$, M and M' are each independently H or an alkali metal element.

Preferably, $R_1$, $R_2$', $R_2$", $R_7$, $R_8$ are each independently H or methyl.

Preferably, M and M' are each independently H or Na.

Preferably, the weight ratio of said structural unit A, said structural unit B, said structural unit C and said structural unit D is 1: (0.02-0.8): (0.005-0.3): (0.005-0.3), more preferably 1: (0.03-0.7): (0.006-0.15): (0.008-0.2).

According to the invention, the polymer has a viscosity average molecular weight within the range of 6,000,000-18,000,000, it may be, for example, 6,500,000, 7,000,000, 7,500,000, 8,000,000, 8,500,000, 9,000,000, 10,000,000, 11,000,000, 12,000,000, 12,500,000, 13,000,000, 13,200,000, 14,000,000, 15,000,000, 17,500,000, and a random value within the range consisting of any two numerical values, preferably within the range of 7,000,000-15,000,000.

According to a more preferred embodiment of the present invention, the polymer comprises a structural unit A, a structural unit B represented by formula (B-1), a structural unit C, and a structural unit D, wherein $R_1$ is H or methyl, $R_2$' is H or methyl, M' is H or an alkali metal (preferably Na), $R_7$ is H, and $R_8$ is H or methyl. In a preferred embodiment, the content of said structural unit A is within the range of 55-87wt% (e.g., 55wt%, 58wt%, 60wt%, 64wt%, 70wt%, 71wt%, 73wt%, 78wt%, 80wt%, 82wt%, 83wt%, 85wt%, or 87wt%), the content of said structural unit B is within the range of 10-25wt% (e.g., 10wt%, 15wt%, 16wt%, 17wt%, 19.5wt%, 20wt%, 22wt%, or 25wt%), the content of said structural unit C is within the range of 1-10wt% (e.g., 1wt%, 1.3wt%, 1.5wt%, 2wt%, 3wt%, 4wt%, 5wt%, 6wt%, 7wt%, 7.5wt%, 8wt%, 9wt%, or 10wt%), and

the content of said structural unit D is within the range of 1-10wt% (e.g., 1wt%, 1.5wt%, 2wt%, 3wt%, 5wt%, 6wt%, 7wt%, 7.5wt%, 8wt%, 9wt%, or 10wt%), based on the total weight of the polymer. The polymer of this preferred embodiment is more suitable for viscosity reduction of heavy oil having a viscosity lower than or equal to 3,000mPa·s at the oil reservoir temperature under the oil reserve conditions consisting of a mineralization degree lower than or equal to 30,000mg/L, a temperature lower than or equal to 70°C, a low driving force (with a rotational speed 20-50r/min).

According to another more preferred embodiment of the present invention, the polymer comprises a structural unit A, a structural unit B represented by formula (B-2), a structural unit C, and a structural unit D, wherein $R_1$ is H or methyl, $R_2$ is $SO_3M$ (

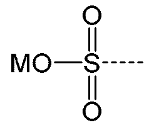

) or $P(O)(OM)_2$ (

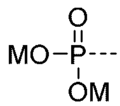

), M is H or an alkali metal (preferably Na), $R_2$" and $R_7$ are H, and $R_8$ is H or methyl. In a preferred embodiment, the content of said structural unit A is within the range of 60-87wt% (e.g., 65wt%, 68wt%, 71wt%, 74wt%, 79wt%, 80wt%, or 85wt%), the content of said structural unit B is within the range of 10-25wt% (e.g., 12wt%, 15wt%, 17.5wt%, 19.5wt%, 20wt%, 22wt%, 23wt%, or 25wt%), the content of said structural unit C is within the range of 1-10wt% (e.g., 1wt%, 1.3wt%, 1.5wt%, 2wt%, 3wt%, 4wt%, 5wt%, 6wt%, 7wt%, 8wt%, 9wt%, or 10wt%), and the content of said structural unit D is within the range of 1-5wt% (e.g., 1wt%, 1.2wt%, 1.3wt%, 1.5wt%, 2wt%, 2.5wt%, 3wt%, 4wt%, or 5wt%), preferably within the range of 1.3-6wt%, more preferably within the range of 1.5-4wt%, based on the total weight of the polymer. The polymer of this preferred embodiment is more suitable for viscosity reduction of heavy oil having a mineralization degree lower than or equal to 50,000mg/L, the concentration of calcium and magnesium ions lower than or equal to 5,000mg/L, a temperature lower than or equal to 70°C, and a viscosity lower than or equal to 3,000mPa·s at the oil reservoir temperature.

The present invention does not impose particular requirements on the specific method for preparing the aforementioned polymer, those skilled in the art can determine a suitable method in accordance with the structural units provided by the present invention in combination with known knowledge in the polymer synthesis field, or those skilled in the art can also prepare the aforementioned polymer by using the specific example (substitute raw material) provided later in the present invention. However, in order to further increase conversion rate of the monomers, the fourth aspect of the present invention provides a method for preparing a glycoside group-containing polymer, is characterized in that the method comprises: subjecting alkenyl monomers in an alkenyl monomer solution to a polymerization reaction in the presence of an initiator under the solution polymerization reaction conditions to obtain a polymer colloid, wherein the alkenyl monomers comprise the compound (monomer C') represented by formula (1), formula (1-1) or formula (1-2).

According to the invention, the content of said compound represented by formula (1) is preferably 0.5wt% or more of the total weight of the alkenyl monomers, more preferably 1-10wt% of the total weight of the alkenyl monomers. Similarly, according to a preferred embodiment of the present invention, the content of said compound represented by formula (1) is 3wt% or more of the total weight of the alkenyl monomers. According to another preferred embodiment of the present invention, the alkenyl monomers comprise the compound represented by formula (1), and the part

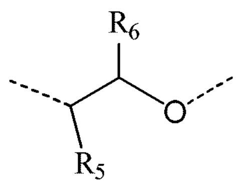

and the part

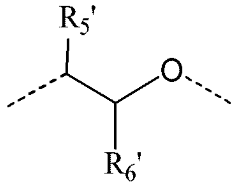

of the compound represented by formula (1) are present simultaneously but are different, and the content of the compound represented by formula (1) is preferably lower than 3wt%, more preferably 1-2.5wt%. The introduction of two specific alcohol ether groups can significantly improve the polymer performance with the lower content of the structural unit provided by the compound.

According to the present invention, the alkenyl monomers can further comprise a monomer represented by formula (3-1) and/or formula (3-2):

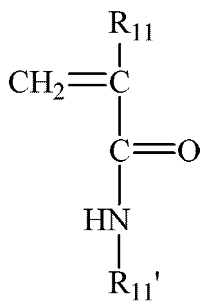 Formula (3-1) 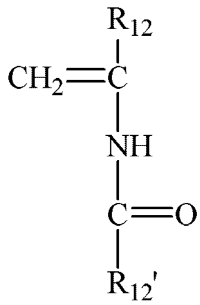 Formula (3-2)

Wherein, $R_{11}$, $R_{12}$, $R_{11}$' and $R_{12}$' are as previously described. The content of said monomer represented by formula (3-1) may be 55wt% or more, preferably 65-97wt% of the total weight of the alkenyl monomers (or the weight ratio of said monomer represented by formula (3-1) to monomer C' is (1-1.8): (0.005-0.3), preferably (1-1.7): (0.006-0.15)). More preferably, the content of said monomer represented by formula (3-2) may be 1-10wt%, preferably 1-5wt% of the total weight of the alkenyl monomers. Preferably, the monomer represented by formula (3-1) is selected from the group consisting of monomer A' represented by formula (a) and/or monomer represented by formula (b-2). Preferably, the monomer represented by formula (3-2) is selected from monomer D' represented by formula (d).

More preferably, the weight ratio of said monomer C' to said monomer D' is (5-300): (5-300), further preferably (6-150): (8-200), most preferably 0.5-10 (e.g., 0.5, 1, 1.5, 2, 2.5, 3, 4, 5, 5.5, 6, 7, 8, 9 or 10).

According to a preferred embodiment of the invention, the alkenyl monomer comprises monomer A' represented by formula (a), monomer B' represented by formula (b-1) or (b-2), monomer C' represented by formula (1) and monomer D' represented by formula (d);

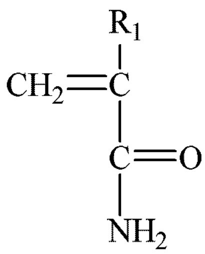 Formula (a), 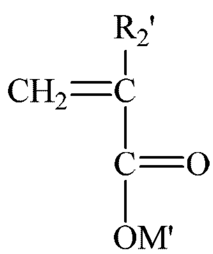 Formula (b-l),

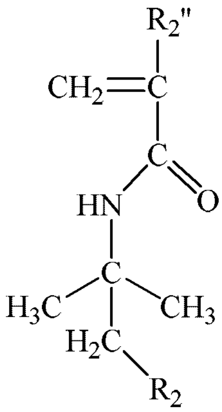 Formula (b-2), 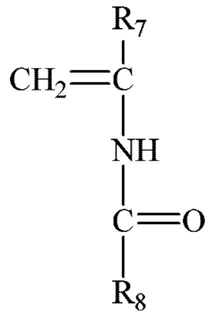 Formula (d);

Wherein, $R_1$, $R_2$, $R_2'$, M', $R_2''$, $R_7$, $R_8$ are as previously described and will not be repeatedly described herein.

Preferably, the weight ratio of said monomer A', monomer B', monomer C' and monomer D' is 1: (0.02-0.8): (0.005-0.3): (0.005-0.3), more preferably 1: (0.03-0.7): (0.006-0.15): (0.008-0.2).

According to a more preferred embodiment of the present invention, the ethylene monomers comprise a monomer A', a monomer B' represented by formula (b-1), a monomer C' and a monomer D', wherein $R_1$ is H or methyl, $R_2'$ is H or methyl, M' is H or an alkali element (preferably Na), $R_7$ is H, and $R_8$ is H or methyl. In a preferred embodiment, the content of said monomer A' is within the range of 55-87wt% (e.g., 55wt%, 58wt%, 60wt%, 64wt%, 70wt%, 71wt%, 73wt%, 78wt%, 80wt%, 82wt%, 83wt%, 85wt%, or 87wt%), the content of said monomer B' is within the range of 10-25wt% (e.g., 10wt%, 15wt%, 16wt%, 17wt%, 19.5wt%, 20wt%, 22wt%, or 25wt%), the content of said monomer C' is within the range of 1-10wt% (e.g., 1wt%, 1.3wt%, 1.5wt%, 2wt%, 3wt%, 4wt%, 5wt%, 6wt%, 7wt%, 7.5wt%, 8wt%, 9wt%, or 10wt%), and the content of said monomer D' is within the range of 1-10wt% (e.g., 1wt%, 1.5wt%, 2wt%, 3wt%, 5wt%, 6wt%, 7wt%, 7.5wt%, 8wt%, 9wt%, or 10wt%), based on the total weight of the polymer.

According to another more preferred embodiment of the present invention, the polymer comprises a monomer A', a monomer B' represented by formula (b-2), a monomer C', and a monomer D', wherein $R_1$ is H or methyl, $R_2$ is $SO_3M$ or $P(O)(OM)_2$, M is H or an alkali metal (preferably Na), $R_2''$ and $R_7$ are H, and $R_8$ is H or methyl. In a preferred embodiment, the content of said monomer A' is within the range of 60-87wt% (e.g., 65wt%, 68wt%, 71wt%, 74wt%, 79wt%, 80wt%, or 85wt%), the content of said monomer B' is within the range of 10-25wt% (e.g., 12wt%, 15wt%, 17.5wt%, 19.5wt%, 20wt%, 22wt%, 23wt%, or 25wt%), the content of said monomer C' is within the range of 1-10wt% (e.g., 1wt%, 1.3wt%, 1.5wt%, 2wt%, 3wt%, 4wt%, 5wt%, 6wt%, 7wt%, 8wt%, 9wt%, or 10wt%), and the content of said monomer D' is within the range of 1-5wt% (e.g., 1wt%, 1.2wt%, 1.3wt%, 1.5wt%, 2wt%, 2.5wt%, 3wt%, 4wt%, or 5wt%), based on the total weight of the alkenyl monomer.

According to the invention, the ratio between the weight of said ethylene monomer and the total weight of solvent and ethylene monomer at the start of the solution polymerization reaction is (0.1-0.4):1, preferably (0.15-0.3):1. The solvent is typically water.

According to the present invention, the solution polymerization conditions cause that the viscosity average molecular weight of the resulting polymer falls within the aforementioned range. Preferably, the solution polymerization conditions comprise: a starting temperature of the polymerization reaction is within the range of -10°C to 30°C, a time of 2-12 hours, and a pH of 4-8. More preferably, the starting temperature of the polymerization reaction is within the range of -5°C to 10°C, the time is within the range of 3-10 hours, and the pH is within the range of 5-7. In the present invention, one or more of an alkali metal hydroxide (e.g., sodium hydroxide, potassium hydroxide), acrylic acid, 2-acrylamido-2-methylpropanesulfonic (phosphonic) acid, may be used for adjusting the pH of said polymerization reaction. It is understandable that by using an alkali metal hydroxide (e.g., sodium hydroxide) for adjusting the pH, M and/or M' in structural unit B (monomer B') can be converted from H to an alkali metal element (e.g., Na), and by controlling the dosage of alkali metal hydroxide, it is also possible to arrange that the polymer of the invention includes both the structural units that M (M') is H and the structural units that M (M') is an alkali metal element (e.g., Na).

According to the invention, the initiator may be selected from various initiators commonly used in the art, for example, the initiator may be selected from an azo-based initiator and/or a redox-based initiator. The initiator may be used in an amount of 0.0001-0.5wt% based on the weight of the ethylene monomer. Preferably, the azo-based initiator is used in an amount of 0.0001-0. 1wt% based on the weight of the ethylene monomer. Preferably, the redox initiator is used in an amount of 0.0002-0.3wt% based on the weight of the ethylene monomer.

According to the present invention, the azo-based initiator is preferably a water-soluble azo-based initiator. The redox initiator includes an oxidizing agent and a reducing agent, the reducing agent is an inorganic reducing agent and/or an organic reducing agent, and the weight ratio of the oxidizing agent to the reducing agent is preferably (0.1-1.2): 1.

Preferably, the water-soluble azo-based initiator is at least one selected from the group consisting of 2,2'-azobis (2-amidinopropane) dihydrochloride, 2'-azobis (2-imidazolidinopropane) dihydrochloride, and 4,4'-azobis (4-cyanovaleric acid). Preferably, the oxidizing agent is at least one selected from the group consisting of benzoyl peroxide, hydrogen peroxide, t-butyl hydrogen peroxide, 2,5-dimethyl-2,5-bis (hydrogen peroxide) hexane, ammonium persulfate, sodium persulfate, and potassium persulfate. Preferably, the inorganic reducing agent is at least one selected from the group consisting of ferrous sulfate, ferrous ammonium sulfate, cuprous chloride, potassium sulfite, sodium sulfite, ammonium bisulfite, potassium bisulfite, sodium thiosulfate, potassium thiosulfate, sodium formaldehyde sulfoxylate (rongalite), and sodium bisulfite. Preferably, the organic reducing agent is at least one selected from the group consisting of N,N-dimethylethanolamine, N,N'-dimethylpiperazine, N,N,N',N'-tetramethylurea, and N,N,N',N'-tetramethylethylenediamine.

According to the invention, in order to desirably control the polymerization induction period and reduce the polymerization inhibition effect of dissolved oxygen, the polymerization reaction is preferably carried out in an inert atmosphere, which may be provided by nitrogen gas and/or an inert gas.

According to the present invention, the process may further comprise: granulating, drying, crushing and sieving the polymer colloid to obtain a polymer product.

According to the present invention, the drying conditions comprise: a temperature of 40-70°C, preferably 45-65°C. In the present invention, the drying time is not particularly limited, the drying may be performed until a solid content of 85-95wt%, preferably a solid content of 88-90wt%, the drying time is generally within the range of 2-24 hours.

The fifth aspect of the present invention provides a glycoside group-containing polymer produced with the aforementioned preparation method.

The functional polymer of the present invention has both a fluidity control function and a spontaneous osmotic emulsification heavy oil function, and can realize strong viscosity reduction of heavy oil having a viscosity lower than or equal to 3,000mPa·s at the oil reservoir temperature under the oil reserve conditions consisting of a mineralization degree lower than or equal to 50,000mg/L (preferably lower than or equal to 30,000mg/L), a concentration of calcium and magnesium ions lower than or equal to 5,000mg/L, a temperature lower than or equal to 70°C, a low driving force (with a rotational speed 20-50r/min); the functional polymer can emulsify and disperse the heavy oil at a low driving force condition, and can be used as a polymer for water flooding heavy oil.

In the present invention, the content of each structural unit in the polymer may be tested by the conventional methods in the prior art, such as Infrared spectroscopy, nuclear magnetism, and the feeding amounts of monomers during polymerization process. Preferably, the content of each structural unit in the polymer is determined by using the feeding amounts of monomers. In particular, the content of each structural unit in the polymer is determined by testing the contents of unreacted monomers to determine the feeding rate of each monomer actually participating in the polymerization. Further, in the present invention, the content of each unreacted monomer in the polymer is tested to be lower than 0.02wt%, it indicates that substantially all of the monomers participate in the polymerization reaction. In particular, the content of residual monomer may be measured by using liquid chromatography.

The sixth aspect of the present invention provides a use of the aforementioned glycoside group-containing polymer in the production of water flooding heavy oil reservoirs. The polymer of the present invention is particularly suitable for the water flooding and exploitation of oil reservoirs having a viscosity lower than or equal to 3,000 mPa·s under the oil reservoir temperature.

In the present invention, water is used as the solvent and reaction medium in the polymer, and the present invention is not particularly limited to its choice. The water may be natural water or artificial water, wherein the natural water may be river water, lake water, atmospheric water, seawater, underground water and the like; the artificial water may be tap water, distilled water, deionized water or heavy water.

Generally speaking, the water used in practical use process will be water at the oilfield site (on-site water) or a corresponding simulated brine. Preferably, the water has a mineralization degree within the range of 1,000-50,000 mg/L (preferably 1,000-30,000 mg/L) and a content of calcium and magnesium ions within the range of 100-5,000mg/L (preferably 100-3,000mg/L).

The seventh aspect of the present invention provides a method for reducing viscosity of heavy oil, is characterized in that the method comprises: contacting a solution of the aforementioned polymer with the heavy oil. The content of polymer in the polymer solution is preferably within the range of 1,000-3,000mg/L. The polymer is preferably used in an amount of 20-50mg (such as 20mg, 25mg, 30mg, 35mg, 40mg, 45mg or 50mg) relative to 70g of the heavy oil.

The eighth aspect of the invention provides a use of the aforementioned compound or polymer in the viscosity reduction of heavy oil (or chemical flooding).

The ninth aspect of the present invention provides a method of exploiting an oil reservoir, is characterized in that the method comprises: applying the aforementioned polymer to an inefficient water flooding oil reservoir, and extracting the heavy oil from the oil reservoir by using the polymer flooding mode to increase the viscosity of the displacement fluid and emulsify the heavy oil. The use mode of the polymer includes dissolving the polymer in a dry powder form in water to obtain a solution with a concentration of 1,000-3,000 mg/L.

The invention will be described in detail below with reference to examples. In the following examples and

Comparative Examples:

Monomer A' was purchased from Dongying Baomo Environmental Engineering Co., Ltd.;
Monomer B' represented by formula (b-1) was purchased from Sigma-Aldrich technology Inc.;

For the monomer B' represented by formula (b-2), when $R_2$ was $SO_3M$, the monomer B' was purchased from Weifang Jinshi Environmental Protection Technology Co., Ltd., when $R_2$ was $P(O)(OM)_2$, the monomer B' was prepared with reference to the synthesis method in the literature "Improved synthesis of AMPP based on Ritter reaction" (HE Quan-guo, SUN Yuan-xi, LIU Ping, et al., INDUSTRIAL WATER TREATMENT, 2001, 21:26-28), when M=Na, the monomer B' was produced from the corresponding monomer of M=H through neutralization with alkali;
The n-hexyl glucoside, decyl glucoside, dodecyl glucoside were commercially available from Guangdong Wengjiang Chemical Reagent Co., Ltd.; the preparation method of alcohol ether glycoside was referred to the literature "Research on Preparation of the Glycosylated Modified Alcohol Ether and Properties thereof" (SUN Gang-jian, YANG Xiu-quan, YANG Qing-li, et al., DETERGENT & COSMETICS, 2007,37:271-274), wherein the molar ratio of glucose to the alcohol ether was controlled at 1:5, the alcohol ether represented by formula (2a) was purchased from Shanghai Jinzijing Chemical Co., Ltd., and the alcohol ether represented by formula (2d) was purchased from Shanghai Banggao Chemical Co., Ltd.

Monomer D' was purchased from Sigma-Aldrich technology Inc.;

Unless otherwise specified in the invention, the reagents and materials used in the examples and Comparative Examples below were commercially available.

In the following examples and Comparative Examples, simulated brines were formulated according to the applicable oil reservoir environment, the simulated brines had a mineralization degree of 30,000mg/L or 50,000mg/L, and a concentration of calcium and magnesium ions of 3,000mg/L or 5,000mg/L (it should be understood that the mineralization degree was an approximate value). Nuclear magnetic analysis was performed by using a Bruker 500MHz nuclear magnetic resonance (NMR) spectrometer.

The test methods for glycoside compound and polymer properties were as follows:

1) The viscosity determination method was referred to the China Petroleum Chemical Enterprise Standard Q/SH 10201957-2008 "Active polymer technology conditions for oil flooding". Instrument: Brookfield DV-III viscometer, rotational speed: 6r/min.
2) The test method of molecular weight was as follows:
The viscosity average molecular weight of polymers was calculated according to the method specified in China National Standard GB/T12005.10-92 using the formula $M=([\eta]/K)^{1/\alpha}$, wherein $K=4.75\times10^{-3}$, $\alpha=0.8$, $[\eta]$ denoted the intrinsic viscosity; the intrinsic viscosity was measured according to the SINOPEC Shengli Petroleum Management Bureau Enterprise Standard Q/SH 10201572-2017 "Polyacrylamide for oil flooding".
3) The viscosity reduction test method was referred to the China Petroleum Chemical Enterprise Standard "Generic technical conditions for heavy oil viscosity reducer" (Q/SH 10201519-2016): the crude oil used in the experiment was an oil sample from an oilfield block in China (The viscosity at 50°C after deaeration on the ground was 1,532mPa·s, 2,389mPa·s, 1,361mPa·s, or 2,620mPa·s). The initial viscosity $\mu_0$ of the test oil sample was determined on a Brookfield DV-III viscometer at 50°C, 70g of crude oil was taken, placed in a 100mL beaker, 30g of functional polymer solution with a concentration of 1,000mg/L was poured, heated at a constant temperature of 50°C for 30min, followed by stirring with a stirrer for 1min at a stirring rate of 20rpm (oil reservoir seepage flow driving force), and the emulsion viscosity $\mu$ was measured. The viscosity reduction rate $f = \frac{\mu_0 - \mu}{\mu_0} \times 100\%$, where: f denoted the viscosity reduction rate, %; $\mu_0$ denoted an initial viscosity of crude oil at 50°C, mPa·s; $\mu$ denoted the viscosity of the crude oil after viscosity reduction, mPa·s.
4) The emulsification properties of glycoside compound were characterized by emulsification time, the specific methods were as follows: the glycoside compound solution with a concentration of 0.1wt% was prepared, 20mL of glycoside compound solution and 20mL of heavy oil (the viscosity at 50°C after deaeration on the ground was 1,532mPa·s) were added into 100mL of a graduated cylinder equipped with a plug, the graduated cylinder was covered with the plug, vigorously vibrated up and down and mixed uniformly then subjected to standing still, the time from start of standing still to the separation of 10mL of aqueous phase was recorded, the emulsification time was tested for 3 times under the same conditions, an average was obtained. The longer the emulsification time, the stronger the emulsifying capability. The test results of emulsification time for glycoside substrates used in the

preparation examples and the modified glycosides obtained were shown in Table 1.

Preparation Example 1.1

The Preparation Example served to illustrate the monomer C' (M1) and its preparation method.

The acrylic acid (130mmol) was taken, then added with toluene (solvent) (300mL), polyoxyethylene ether polyoxypropylene ether alcohol ether glycosides (100mmol, the structure was as shown in formula (2d), wherein $R_3$ was 2-ethylhexyl, R5' was methyl, $R_5$, $R_6$ and $R_6$' were H, n1 was 3, n2 was 6), and triflic acid (13mmol) at room temperature; after the materials were completely added, the materials were heated to 120°C and subjected to reflux and water division, the reaction was performed for 8h; after the raw materials were completely reacted, the reactants were cooled to room temperature, both the toluene and acid were removed through the reduced pressure distillation, the product acrylic acid-based modified alcohol ether glycoside was obtained. The product yield was 72.5%, and the purity was 92.3%. The purity and the yield were calculated by using the nuclear magnetic internal standard method, the calculation formula of said yield = the actual production of the target product / production amount of target product $\times$100%, the calculation formula of said purity = the calculated production of nuclear magnetic resonance / actual charge weight of nuclear magnetic resonance $\times$100% (hereinafter the same).

Upon nuclear magnetic analysis, the product structure was as shown by formula (1), wherein $R_3$ was 2-ethylhexyl, $R_4$ was H, X was O, $R_5$' was methyl, $R_5$, $R_6$ and $R_6$' were H, n1 was 3, and n2 was 6.

The specific nuclear magnetic data was as follows: 6.40 (dd, *J* = 19.8, 4.6 Hz, 1H), 6.12 (dd, *J* = 33.5, 19.8 Hz, 1H), 5.82 (dd, *J* = 33.5, 4.6 Hz, 1H), 5.35 - 5.20 (m, 1H), 4.77 (dd, *J* = 24.2, 12.3 Hz, 1H), 4.29 - 3.86 (m, 7H), 3.84 - 3.30 (m, 32H), 3.16 - 3.05 (m, 1H), 1.86 (s, 1H), 1.62 - 1.35 (m, 6H), 1.33 - 1.29 (m, 10H), 1.29 - 1.20 (m, 4H), 0.98 - 0.85 (m, 6H).

Preparation Example 1.2

The Preparation Example served to illustrate the monomer C' (M2) and its preparation method.

The acrylic acid (90mmol) was taken, then added with toluene (300mL), polyoxyethylene ether polyoxypropylene ether alcohol ether glycosides (100mmol, the structure was as shown in formula (2d), wherein $R_3$ was n-C12 alkyl, R5' was methyl, $R_5$, $R_6$ and $R_6$' were H, n1 was 6, n2 was 9) and triflic acid (20mmol) at room temperature; after the materials were completely added, the materials were heated to 140°C and subjected to reflux and water division, the reaction was performed for 10h; after the raw materials were completely reacted, the reactants were cooled to room temperature, both the toluene and acid were removed through the reduced pressure distillation, the product acrylic acid-based modified alcohol ether glycoside was obtained. The product yield was 61.5%, and the purity was 93.1%.

Upon nuclear magnetic analysis, the product structure was as shown by formula (1), wherein $R_3$ was n-C12 alkyl, $R_4$ was H, X was O, $R_5$' was methyl, $R_5$, $R_6$ and $R_6$' were H, n1 was 6, and n2 was 9.

Preparation Example 1.3

The Preparation Example served to illustrate the monomer C' (M3) and its preparation method.

The triphenylphosphine activator (150mmol) and the polyoxyethylene ether polyoxypropylene ether alcohol ether glycoside (100mmol, the structure was as shown in formula (2d), wherein $R_3$ was 11-methyldodecyl, $R_5$' was methyl, $R_5$, $R_6$ and $R_6$' were H, n1 was 2, n2 was 11) were taken, the nitrogen gas was introduced for removing oxygen gas, N,N-dimethylformamide solvent (300mL) and methacrylic acid (120mmol) were then added, the temperature was lowered to 0°C, and the diisopropyl azodicarboxylate activator (150mmol) was slowly and dropwise added; after the materials were completely added, the temperature was raised to room temperature 25°C, the reaction was performed for 8h; after completion of the reaction, the solvent was removed through the reduced pressure distillation, the crude product was separated by column chromatography (volume ratio of dichloromethane to methanol = 9:1), the methacrylate-based modified glycoside was obtained. The product yield was 65.5%, and the purity was 90.2%.

After the nuclear magnetic detection, $R_3$ was 11-methyldodecyl, in particular, upon nuclear magnetic analysis, the product structure was as shown by formula (1), wherein $R_3$ was 11-methyldodecyl, $R_4$ was methyl, X was O, $R_5$' was methyl, $R_5$, $R_6$ and $R_6$' were H, n1 was 2, and n2 was 11.

Preparation Example 1.4

The Preparation Example served to illustrate the monomer C' (M4) and its preparation method.

The acrylic acid (130mmol) was taken, then added with toluene (solvent) (300mL), n-hexyl glucoside (100mmol) and triflic acid (39mmol) at room temperature; after the materials were completely added, the materials were heated to 120°C and subjected to reflux and water division, the reaction was performed for 8h; after the raw materials were completely reacted, the reactants were cooled to room temperature, both the toluene and acid were removed through the

reduced pressure distillation, the product acrylic acid-based modified glycoside was obtained. The product yield was 75.1%, and the purity was 91.9%.

Upon nuclear magnetic analysis, the product structure was as shown by formula (1-1), wherein $R_3$ was n-hexyl, $R_4$ was H, and n =0.

The nuclear magnetic data was as follows: $^1$H NMR (300 MHz, DMSO-$d_6$) $\delta$ : 6.41 (dd, *J* = 19.9, 4.5 Hz, 1H), 6.12 (dd, *J* = 33.5, 19.8 Hz, 1H), 5.83 (dd, *J* = 33.5, 4.4 Hz, 1H), 4.60 (d, *J* = 15.4 Hz, 1H), 4.45 (dd, *J* = 24.8, 3.4 Hz, 1H), 4.21 (dd, *J* = 24.8, 3.4 Hz, 1H), 4.12 - 3.91 (m, 2H), 3.58 (dd, *J* = 17.5, 16.4 Hz, 1H), 3.48 (dt, *J* = 18.6, 3.4 Hz, 1H), 3.39 - 3.32 (m, 2H), 1.63 (s, 3H), 1.59 - 1.31 (m, 8H), 0.94 - 0.84 (m, 3H).

Preparation Example 1.5

The Preparation Example served to illustrate the monomer C' (M5) and its preparation method.

The acrylic acid (90mmol) was taken, then added with toluene (300mL), dodecyl glucoside (100mmol) and triflic acid (18mmol) at room temperature; after the materials were completely added, the materials were heated to 140°C and subjected to reflux and water division, the reaction was performed for 10h; after the raw materials were completely reacted, the reactants were cooled to room temperature, both the toluene and acid were removed through the reduced pressure distillation, the product acrylic acid-based modified glycoside was obtained. The product yield was 58.0%, and the purity was 93.1%.

Upon nuclear magnetic analysis, the product structure was as shown by formula (1-1), wherein $R_3$ was n-dodecyl, $R_4$ was H, and n =0.

Preparation Example 1.6

The Preparation Example served to illustrate the monomer C' (M6) and its preparation method.

The triphenylphosphine activator (150mmol) and n-decyl glucoside (100mmol) were taken, after the nitrogen gas was introduced for removing oxygen gas, N,N-dimethylformamide solvent (300mL) and methacrylic acid (120mmol) were added; the temperature was lowered to 0°C, and the diisopropyl azodicarboxylate activator (150mmol) was slowly and dropwise added; after the materials were completely added, the temperature was raised to room temperature 25°C, the reaction was performed for 8h; after completion of the reaction, the solvent was removed through the reduced pressure distillation, the crude product was separated by column chromatography (volume ratio of dichloromethane to methanol = 9:1), the methacrylate-based modified glucoside was obtained. The product yield was 69.8%, and the purity was 91.8%.

Upon nuclear magnetic analysis, the product structure was as shown by formula (1-1), wherein $R_3$ was n-decyl, $R_4$ was methyl, and n =0.

Preparation Example 1.7

The Preparation Example served to illustrate the monomer C' (M7) and its preparation method.

The acrylic acid (130mmol) was taken, then added with toluene (300mL), alcohol ether glycosides (100mmol, the structure was as shown in formula (2a), wherein $R_3$ was straight chain C8 alkyl and straight chain C10 alkyl, n was 8) and triflic acid (20mmol) at room temperature; after the materials were completely added, the materials were heated to 120°C and subjected to reflux and water division, the reaction was performed for 7h; after the raw materials were completely reacted, the reactants were cooled to room temperature, both the toluene and acid were removed through the reduced pressure distillation, the product acrylic acid-based modified glycoside was obtained. The product yield was 82.9%, and the purity was 90.6%.

Upon nuclear magnetic analysis, the product structure was as shown by formula (1-1), wherein $R_3$ was straight chain C8 alkyl and straight chain C10 alkyl, $R_4$ was H, and n was 8.

$^1$H NMR (300 MHz, DMSO-$d_6$) $\delta$ 6.41 (dd, *J* = 19.9, 4.5 Hz, 2H), 6.12 (dd, *J* = 33.5, 19.8 Hz, 2H), 5.83 (dd, *J* = 33.5, 4.4 Hz, 2H), 4.59 (dd, *J* = 24.8, 13.8 Hz, 2H), 4.52 (d, *J* = 15.4 Hz, 2H), 4.18 - 4.03 (m, 4H), 3.77 (dd, *J* = 18.3, 16.2 Hz, 2H), 3.60 - 3.46 (m, 33+37H), 3.44 - 3.28 (m, 6H), 1.65 - 1.39 (m, 14H), 1.34 - 1.20 (m, 16H), 0.93 - 0.85 (m, 6H).

Preparation Example 1.8

The Preparation Example served to illustrate the monomer C' (M8) and its preparation method.

The acrylic acid (150mmol) was taken, then added with toluene (300mL), alcohol ether glycosides (100mmol, the structure was as shown in formula (2a), wherein $R_3$ was straight chain C12 alkyl and straight chain C14 alkyl, n was 4) and triflic acid (15mmol) at room temperature; after the materials were completely added, the materials were heated to 140°C and subjected to reflux and water division, the reaction was performed for 10h; after the raw materials were completely reacted, the reactants were cooled to room temperature, both the toluene and acid were removed through the reduced

pressure distillation, the product acrylic acid-based modified glycoside was obtained. The product yield was 70.2%, and the purity was 87.7%.

Upon nuclear magnetic analysis, the product structure was as shown by formula (1-1), wherein $R_3$ was straight chain C12 alkyl and straight chain C14 alkyl, $R_4$ was H, and n was 4.

Preparation Example 1.9

The Preparation Example served to illustrate the monomer C' (M9) and its preparation method.

The triphenylphosphine activator (150mmol) and the alcohol ether glycoside (100mmol, the structure was as shown in formula (2d), wherein $R_3$ was straight chain C12 alkyl and straight chain C14 alkyl, n was 7), the nitrogen gas was introduced for removing oxygen gas, N,N-dimethylformamide solvent (300mL) and acrylic acid (120mmol) were then added, the temperature was lowered to 0°C, and the diisopropyl azodicarboxylate (150mmol) was slowly and dropwise added; after the materials were completely added, the temperature was raised to room temperature 25°C, the reaction was performed for 8h; after completion of the reaction, the solvent was removed through the reduced pressure distillation, the crude product was separated by column chromatography (volume ratio of dichloromethane to methanol = 5:1), the acrylic acid -based modified glycoside was obtained. The product yield was 71.3%, and the purity was 94.6%.

Upon nuclear magnetic analysis, the product structure was as shown by formula (1-1), wherein $R_3$ was straight chain C12 alkyl and straight chain C14 alkyl, $R_4$ was H, and n was 7.

Preparation Example 1.10

The monomers were prepared with the same method as in Preparative Example 1.4, except that the acrylic acid was replaced with an equivalent amount of maleic anhydride, the maleic anhydride-modified alkyl glycoside (M10) was obtained.

Preparation Example 1.11

The monomers were prepared with the same method as in Preparative Example 1.1, except that the structure of polyoxyethylene ether polyoxypropylene ether alcohol ether glycoside in use was as shown in formula (2d), wherein $R_3$ was 2-ethylhexyl, $R_5$ was methyl, $R_6$, $R_5$' and $R_6$' were H, n1 was 3, n2 was 6, the acrylic acid-based modified alcohol ether glycoside (M11) was obtained.

Preparation Example 2.1

The Preparation Example served to illustrate the monomer C' (N1) and its preparation method.

(1) The polyoxyethylene ether polyoxypropylene ether alcohol ether glycosides (100mmol, the structure was as shown in formula (2d), wherein $R_3$ was 2-ethylhexyl, R5' was methyl, $R_5$, $R_6$ and $R_6$' were H, n1 was 3, n2 was 6) were taken, nitrogen gas was then introduced for removing oxygen gas, cooled to 0°C, trifluoromethanesulfonic anhydride (90mmol) and pyridine (3,727mmol) were added, after the materials were completely added, the materials were kept at the temperature of 0°C and subjected to the reaction for 6h; after the raw materials were reacted completely, the temperature was recovered to room temperature, water was added for quenching the reaction, dichloromethane was used for extraction three times, the organic phases were combined and subjected to drying with anhydrous magnesium sulfate; the solvent and pyridine were subsequently removed through the reduced pressure distillation, the product alcohol ether glycoside sulfonate ester was obtained. The product yield was 45.2%, and the purity was 65.2%.

(2) The acrylamide (150mmol) was taken, added with alcohol ether glycoside sulfonate ester (100mmol); nitrogen gas was then introduced for removing oxygen gas, tetrahydrofuran (300mL) and sodium hydride (200mmol) were added at room temperature; after the materials were completely added, the materials were heated to 80°C and subjected to reflux, the reaction was performed for 6h; after the raw materials were completely reacted, cooled to room temperature, the saturated ammonium chloride solution was added for quenching the reaction, dichloromethane was used for extraction three times, the organic phases were combined and subjected to drying with anhydrous magnesium sulfate; the solvent and pyridine were subsequently removed through the reduced pressure distillation, the product acrylamide-based modified glycoside was obtained. The product yield was 70.1%, and the purity was 89.5%.

Upon nuclear magnetic analysis, the product structure was as shown by formula (1), wherein $R_3$ was 2-ethylhexyl, $R_4$ was H, X was NH, $R_5$' was methyl, $R_5$, $R_6$ and $R_6$' were H, n1 was 3, and n2 was 6. The specific nuclear

magnetic data was as follows: $^1H$ NMR (300 MHz, DMSO-$d_6$) δ 6.19 (dd, $J$ = 33.0, 20.0 Hz, 1H), 6.08 - 6.01 (m, 2H), 5.69 (dd, $J$ = 33.0, 4.9 Hz, 1H), 4.91 (dd, $J$ = 3.6, 1.8 Hz, 1H), 4.77 (d, $J$ = 1.8 Hz, 1H), 4.32 (dd, $J$ = 8.6, 3.6 Hz, 1H), 4.00 - 3.76 (m, 5H), 3.73 - 3.61 (m, 3H), 3.57 - 2.95 (m, 32H), 1.76 (s, 1H), 1.59 - 1.48 (m, 3H), 1.45 - 1.20 (m, 16H), 0.96 - 0.85 (m, 6H).

Preparation Example 2.2

The Preparation Example served to illustrate the monomer C' (N2) and its preparation method.

(1) The polyoxyethylene ether polyoxypropylene ether alcohol ether glycosides (100mmol, the structure was as shown in formula (2d), wherein $R_3$ was n-C12 alkyl, R5' was methyl, $R_5$, $R_6$ and $R_6$' were H, n1 was 6, n2 was 9) were taken, nitrogen gas was then introduced for removing oxygen gas, trifluoromethanesulfonic anhydride (120mmol) and pyridine (3,727mmol) were added, after the materials were completely added, the materials were kept at the room temperature and subjected to the reaction for 6h; after the raw materials were reacted completely, water was added for quenching the reaction, dichloromethane was used for extraction three times, the organic phases were combined and subjected to drying with anhydrous magnesium sulfate; the solvent and pyridine were subsequently removed through the reduced pressure distillation, the product alcohol ether glycoside sulfonate ester was obtained. The product yield was 48.5%, and the purity was 72.3%.

(2) The acrylamide (200mmol) was taken, added with alcohol ether glycoside sulfonate ester (100mmol); nitrogen gas was then introduced for removing oxygen gas, tetrahydrofuran (300mL) and sodium hydride (200mmol) were added at room temperature; after the materials were completely added, the materials were heated to 100°C and subjected to reflux, the reaction was performed for 8h; after the raw materials were completely reacted, cooled to room temperature, the saturated ammonium chloride solution was added for quenching the reaction, dichloromethane was used for extraction three times, the organic phases were combined and subjected to drying with anhydrous magnesium sulfate; the solvent and pyridine were subsequently removed through the reduced pressure distillation, the product acrylamide-based modified glycoside was obtained. The product yield was 74.2%, and the purity was 90%.

Upon nuclear magnetic analysis, the product structure was as shown by formula (1), wherein $R_3$ was n-C12 alkyl, $R_4$ was H, X was NH, $R_5$' was methyl, $R_5$, $R_6$ and $R_6$' were H, n1 was 6, and n2 was 9.

Preparation Example 2.3

The Preparation Example served to illustrate the monomer C' (N3) and its preparation method.

The triphenylphosphine activator (150mmol) was taken, the polyoxyethylene ether polyoxypropylene ether alcohol ether glycosides (100mmol, the structure was as shown in formula (2d), wherein $R_3$ was 11-methyl dodecyl, R5' was methyl, $R_5$, $R_6$ and $R_6$' were H, n1 was 2, n2 was 11) were taken, nitrogen gas was then introduced for removing oxygen gas, N,N-dimethylformamide solvent (3,894mmol) and methacrylamide (120mmol) were subsequently added, cooled to 0°C, the diisopropyl azodicarboxylate activator (150mmol) was slowly and dropwise added; after the materials were completely added, the materials were heated to the room temperature 25°C and subjected to the reaction for 8h; after completion of the reaction, the solvent was removed through the reduced pressure distillation, the crude product was separated by column chromatography (volume ratio of dichloromethane to methanol = 9:1), the acrylamide-based modified glycoside was obtained. The product yield was 58.1%, and the purity was 91.0%.

Upon nuclear magnetic analysis, the product structure was as shown by formula (1), wherein $R_3$ was 11-methyl dodecyl, $R_4$ was methyl, X was NH, $R_5$' was methyl, $R_5$, $R_6$ and $R_6$' were H, n1 was 2, and n2 was 11.

Preparation Example 2.4

The Preparation Example served to illustrate the monomer C' (N4) and its preparation method.

(1) The n-hexyl glucoside (100mmol) was taken, nitrogen gas was then introduced for removing oxygen gas, cooled to 0°C, p-toluenesulfonyl chloride (90mmol) and pyridine (3727mmol) were added, after the materials were completely added, the materials were kept at the temperature of 0°C and subjected to the reaction for 6h; after the raw materials were reacted completely, the temperature was recovered to room temperature, water was added for quenching the reaction, dichloromethane was used for extraction three times, the organic phases were combined and subjected to drying with anhydrous magnesium sulfate; the solvent and pyridine were subsequently removed through the reduced pressure distillation, the product glucoside sulfonate ester was obtained. The product yield was 42.3%, and the purity was 60.8%.

(2) The acrylamide (150mmol) was taken, added with glucoside sulfonate ester (100mmol); nitrogen gas was then

introduced for removing oxygen gas, tetrahydrofuran (300mL) and sodium hydride (200mmol) were added at room temperature; after the materials were completely added, the materials were heated to 80°C and subjected to reflux, the reaction was performed for 6h; after the raw materials were completely reacted, cooled to room temperature, the saturated ammonium chloride solution was added for quenching the reaction, dichloromethane was used for extraction three times, the organic phases were combined and subjected to drying with anhydrous magnesium sulfate; the solvent and acrylamide were subsequently removed through the reduced pressure distillation, the product acrylamide-based modified glucoside was obtained. The product yield was 68.9%, and the purity was 95.6%.

Upon nuclear magnetic analysis, the product structure was as shown by formula (1-2), wherein $R_3$ was n-hexyl, $R_4$ was H, n=0. $^1$H NMR (300 MHz, DMSO-$d_6$) δ 6.12 (s, 1H), 6.05 - 5.92 (m, 2H), 5.69 (dd, *J* = 30.8, 7.1 Hz, 1H), 4.56 (d, *J* = 15.2 Hz, 1H), 4.02 - 3.94 (m, 1H), 3.62 (dq, *J* = 32.7, 16.7 Hz, 2H), 3.48 (dt, *J* = 18.3, 5.9 Hz, 1H), 3.40 - 3.33 (m, 2H), 3.27 (dd, *J* = 18.9, 5.9 Hz, 1H), 3.02 (dd, *J* = 24.8, 5.8 Hz, 1H), 1.88 (s, 3H), 1.57 - 1.30 (m, 8H), 0.89 (s, 3H).

The reaction process of the Preparation Example was shown as follows:

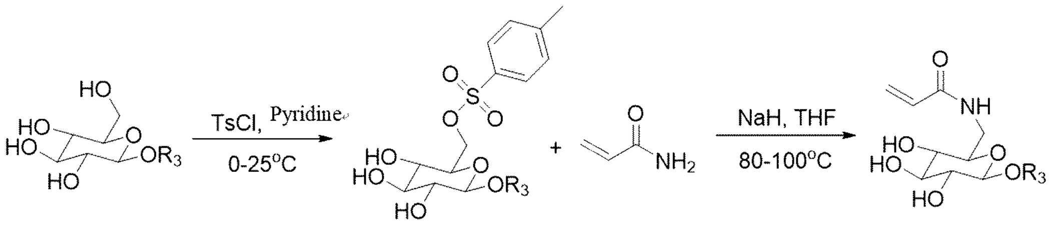


Preparation Example 2.5

The Preparation Example served to illustrate the monomer C' (N5) and its preparation method.

(1) The dodecyl glucoside (100mmol) was taken, nitrogen gas was then introduced for removing oxygen gas, p-toluenesulfonyl chloride (120mmol) and pyridine (3,727mmol) were added, after the materials were completely added, the materials were kept at the room temperature and subjected to the reaction for 6h; after the raw materials were reacted completely, water was added for quenching the reaction, dichloromethane was used for extraction three times, the organic phases were combined and subjected to drying with anhydrous magnesium sulfate; the solvent and pyridine were subsequently removed through the reduced pressure distillation, the product glucoside sulfonate ester was obtained. The product yield was 69.3%, and the purity was 92.3%.

(2) The acrylamide (200mmol) was taken, added with glucoside sulfonate ester (100mmol); nitrogen gas was then introduced for removing oxygen gas, tetrahydrofuran (300mL) and sodium hydride (200mmol) were added at room temperature; after the materials were completely added, the materials were heated to 100°C and subjected to reflux, the reaction was performed for 8h; after the raw materials were completely reacted, cooled to room temperature, the saturated ammonium chloride solution was added for quenching the reaction, dichloromethane was used for extraction three times, the organic phases were combined and subjected to drying with anhydrous magnesium sulfate; the solvent and acrylamide were subsequently removed through the reduced pressure distillation, the product acrylamide-based modified glucoside was obtained. The product yield was 71.3%, and the purity was 96.9%.

Upon nuclear magnetic analysis, the product structure was as shown by formula (1-2), wherein $R_3$ was n-dodecyl, $R_4$ was H, n=0.

Preparation Example 2.6

The Preparation Example served to illustrate the monomer C' (N6) and its preparation method.

The triphenylphosphine activator (150mmol) and N-decyl glucoside (100mmol) were taken, nitrogen gas was then introduced for removing oxygen gas, the N,N-dimethylformamide solvent (3,894mmol) and methacrylamide (120mmol) were added, cooled to 0°C, the diisopropyl azodicarboxylate activator (150mmol) was slowly and dropwise added; after the materials were completely added, the materials were heated to the room temperature 25°C and subjected to the reaction for 8h; after completion of the reaction, the solvent was removed through the reduced pressure distillation, the crude product was separated by column chromatography (volume ratio of dichloromethane to methanol = 9:1), the acrylamide-based modified glucoside was obtained. The product yield was 53.8%, and the purity was 91.8%.

Upon nuclear magnetic analysis, the product structure was as shown by formula (1-2), wherein $R_3$ was n-decyl, $R_4$ was methyl, and n=0.

Preparation Example 2.7

The Preparation Example served to illustrate the monomer C' (N7) and its preparation method.

(1) The alcohol ether glycosides (100mmol, the structure was as shown in formula (2a), wherein $R_3$ was straight chain C8 alkyl and straight chain C10 alkyl, n was 10) were taken, nitrogen gas was then introduced for removing oxygen gas, cooled to 0°C, p-toluene sulfonyl chloride (90mmol) and pyridine (3,727mmol) were added, after the materials were completely added, the materials were kept at the temperature of 0°C and subjected to the reaction for 6h; after the raw materials were reacted completely, the temperature was recovered to room temperature, water was added for quenching the reaction, dichloromethane was used for extraction three times, the organic phases were combined and subjected to drying with anhydrous magnesium sulfate; the solvent and pyridine were subsequently removed through the reduced pressure distillation, the product alcohol ether glycoside sulfonate ester was obtained. The product yield was 49.3%, and the purity was 72.9%.

(2) The acrylamide (150mmol) was taken, added with alcohol ether glycoside sulfonate ester (100mmol); nitrogen gas was then introduced for removing oxygen gas, tetrahydrofuran (300mL) and sodium hydride (200mmol) were added at room temperature; after the materials were completely added, the materials were heated to 80°C and subjected to reflux, the reaction was performed for 8h; after the raw materials were completely reacted, cooled to room temperature, the saturated ammonium chloride solution was added for quenching the reaction, dichloromethane was used for extraction three times, the organic phases were combined and subjected to drying with anhydrous magnesium sulfate; the solvent and pyridine were subsequently removed through the reduced pressure distillation, the product acrylamide-based modified alcohol ether glycoside was obtained. The product yield was 59.7%, and the purity was 91.2%.

Upon nuclear magnetic analysis, the product structure was as shown by formula (1-2), wherein $R_3$ was straight chain C8 alkyl and straight chain C10 alkyl, $R_4$ was H, and n was 10.

$^1$H NMR (300 MHz, DMSO-$d_6$) δ 6.11 - 6.03 (m, 4H), 5.99 (s, 2H), 5.69 (dd, *J* = 14.4, 4.5 Hz, 2H), 4.60 (d, *J* = 7.7 Hz, 2H), 4.12 - 4.06 (m, 2H), 3.85 - 3.79 (m, 2H), 3.62 (t, *J* = 8.5 Hz, 2H), 3.59 - 3.48 (m, 45+49H), 3.33 (t, *J* = 7.3 Hz, 4H), 3.27 (dd, *J* = 12.5, 6.2 Hz, 2H), 3.02 (dd, *J* = 12.5, 6.2 Hz, 2H), 1.74 (s, 6H), 1.58 - 1.43 (m, 8H), 1.41 - 1.33 (m, 8H), 0.92 - 0.86 (m, 6H).

Preparation Example 2.8

The Preparation Example served to illustrate the monomer C' (N8) and its preparation method.

(1) The alcohol ether glycosides (100mmol, the structure was as shown in formula (2a), wherein $R_3$ was straight chain C12 alkyl and straight chain C14 alkyl, n was 4) were taken, nitrogen gas was then introduced for removing oxygen gas, p-toluene sulfonyl chloride (120mmol) and pyridine (3,727mmol) were added, after the materials were completely added, the materials were kept at the room temperature and subjected to the reaction for 8h; after the raw materials were reacted completely, water was added for quenching the reaction, dichloromethane was used for extraction three times, the organic phases were combined and subjected to drying with anhydrous magnesium sulfate; the solvent and pyridine were subsequently removed through the reduced pressure distillation, the product alcohol ether glycoside sulfonate ester was obtained. The product yield was 69.8%, and the purity was 90.1%.

(2) The acrylamide (100mmol) was taken, added with alcohol ether glycoside sulfonate ester (100mmol); nitrogen gas was then introduced for removing oxygen gas, tetrahydrofuran (300mL) and sodium hydride (200mmol) were added at room temperature; after the materials were completely added, the materials were heated to 90°C and subjected to reflux, the reaction was performed for 9.5h; after the raw materials were completely reacted, cooled to room temperature, the saturated ammonium chloride solution was added for quenching the reaction, dichloromethane was used for extraction three times, the organic phases were combined and subjected to drying with anhydrous magnesium sulfate; the solvent and acrylamide were subsequently removed through the reduced pressure distillation, the product acrylamide-based modified alcohol ether glycoside was obtained. The product yield was 72.3%, and the purity was 97.1%.

Upon nuclear magnetic analysis, the product structure was as shown by formula (1-2), wherein $R_3$ was straight chain C12 alkyl and straight chain C14 alkyl, $R_4$ was H, and n was 4.

Preparation Example 2.9

The Preparation Example served to illustrate the monomer C' (N9) and its preparation method.

(1) The alcohol ether glycosides (100mmol, the structure was as shown in formula (2a), wherein $R_3$ was straight chain C12 alkyl and straight chain C14 alkyl, n was 7) were taken, nitrogen gas was then introduced for removing oxygen gas, p-toluene sulfonyl chloride (150mmol) and pyridine (3,727mmol) were added, after the materials were completely added, the materials were kept at the room temperature and subjected to the reaction for 8h; after the raw materials were reacted completely, water was added for quenching the reaction, dichloromethane was used for extraction three times, the organic phases were combined and subjected to drying with anhydrous magnesium sulfate; the solvent and pyridine were subsequently removed through the reduced pressure distillation, the product alcohol ether glycoside sulfonate ester was obtained. The product yield was 75.3%, and the purity was 86.9%.
(2) The acrylamide (200mmol) was taken, added with alcohol ether glycoside sulfonate ester (100mmol); nitrogen gas was then introduced for removing oxygen gas, tetrahydrofuran (300mL) and sodium hydride (200mmol) were added at room temperature; after the materials were completely added, the materials were heated to 80°C and subjected to reflux, the reaction was performed for 6h; after the raw materials were completely reacted, cooled to room temperature, the saturated ammonium chloride solution was added for quenching the reaction, dichloromethane was used for extraction three times, the organic phases were combined and subjected to drying with anhydrous magnesium sulfate; the solvent and acrylamide were subsequently removed through the reduced pressure distillation, the product acrylamide-based modified alcohol ether glycoside was obtained. The product yield was 65.3%, and the purity was 88.1%.

Upon nuclear magnetic analysis, the product structure was as shown by formula (1-2), wherein $R_3$ was straight chain C12 alkyl and straight chain C14 alkyl, $R_4$ was H, and n was 7.

Preparation Example 2.10

The Preparation Example served to illustrate the monomer C' (N10) and its preparation method.
The triphenylphosphine (150mmol) and the alcohol ether glycoside (100mmol, the structure was as shown in formula (2a), wherein $R_3$ was straight chain C8 alkyl and straight chain C10 alkyl, n was 5), the nitrogen gas was introduced for removing oxygen gas, N,N-dimethylformamide solvent (3,894mL) and acrylamide (130mmol) were then added, the temperature was lowered to 0°C, and the diisopropyl azodicarboxylate (150mmol) was slowly and dropwise added; after the materials were completely added, the temperature was raised to room temperature 25°C, the reaction was performed for 8h; after completion of the reaction, the solvent was removed through the reduced pressure distillation, the crude product was separated by column chromatography (volume ratio of dichloromethane to methanol = 8:1), the acrylamide-based modified alcohol ether glycoside was obtained. The product yield was 52.9%, and the purity was 94.6%.
Upon nuclear magnetic analysis, the product structure was as shown by formula (1-2), wherein $R_3$ was straight chain C8 alkyl and straight chain C10 alkyl, $R_4$ was H, and n was 5.
The reaction process of the Preparation Example was shown as follows:

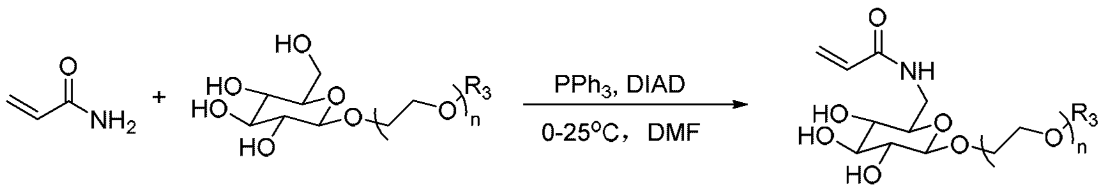


Preparation Example 2.11

The monomers were prepared with the same method as in Preparative Example 2.1, except that the polyoxyethylene ether polyoxypropylene ether alcohol ether glycoside in use had a structure represented by formula (2d), wherein $R_3$ was 2-ethyl hexyl, $R_5$ was methyl, $R_6$, $R_5$' and $R_6$' were reach H, n1 was 3, n2 was 6 (N11).

Table 1 Emulsification time test results for glycoside group-containing compound

| No. | Emulsification time (min) | | No. | Emulsification time (min) | |
|---|---|---|---|---|---|
| | Glycoside substrate | After modification | | Glycoside substrate | After modification |
| Preparation Example 1.1 | 17.4 | 18.9 | Preparation Example 2.1 | - | 19.2 |

(continued)

| No. | Emulsification time (min) | | No. | Emulsification time (min) | |
|---|---|---|---|---|---|
| | Glycoside substrate | After modification | | Glycoside substrate | After modification |
| Preparation Example 1.2 | 17.2 | 18.5 | Preparation Example 2.2 | - | 18.4 |
| Preparation Example 1.3 | 16.9 | 18.3 | Preparation Example 2.3 | - | 18.6 |
| Preparation Example 1.4 | 12.1 | 13.6 | Preparation Example 2.4 | - | 13.4 |
| Preparation Example 1.5 | 12.9 | 14.3 | Preparation Example 2.5 | - | 14.2 |
| Preparation Example 1.6 | 12.4 | 14.1 | Preparation Example 2.6 | - | 14.4 |
| Preparation Example 1.7 | 14.6 | 16.1 | Preparation Example 2.7 | 14.5 | 16.3 |
| Preparation Example 1.8 | 16.0 | 17.6 | Preparation Example 2.8 | - | 17.5 |
| Preparation Example 1.9 | 15.5 | 16.7 | Preparation Example 2.9 | - | 16.9 |
| Preparation Example 1.10 | 12.1 | 12.5 | Preparation Example 2.10 | 16.8 | 18.0 |
| Preparation Example 1.11 | 15.9 | 17.5 | Preparation Example 2.11 | - | 17.8 |

By reacting the compound represented by formula (3) with the compound represented by formula (2), the emulsifying properties of the compound represented by formula (2) to the heavy oil can be significantly improved, and the improved effect of the compound represented by formula (3) is superior to that of maleic anhydride.

Example 1.1

(1) 46.32g of monomer A', 11.28g of monomer B', 1.2g of monomer C' (M1) and 1.2g of monomer D' were taken, wherein the structure of said monomer A' was as shown in formula (a) (wherein $R_1$ was H);

The structure of said monomer B' was as shown in formula (b-1) (wherein $R_2$' was H, M' was Na);
The structure of said monomer D' was as shown in formula (d) (wherein $R_7$ and $R_8$ were each H);
The monomers were dissolved in 240g of deionized water, sodium hydroxide solution was used for adjusting the pH to 6, the starting temperature was controlled to 10°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate having a concentration of 0.2wt%, and 1.25g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (its structure was as shown in formula (A), wherein $R_1$ was H);
Structural unit B (its structure was as shown in formula (B-1), wherein $R_2$' was H, M' was Na);
Structural unit C (its structure was as shown in formula (C), wherein $R_3$ was 2-ethylhexyl, $R_4$ was H, X was O, $R_5$' was methyl, $R_5$, $R_6$ and $R_6$' were each H, n1 was 3, n2 was 6);
Structural unit D (its structure was as shown in formula (D), wherein $R_7$ and $R_8$ were each H);

Wherein the structural unit A was contained in an amount of 77.2wt%, the structural unit B was contained in an amount of 18.8wt%, the structural unit C was contained in an amount of 2wt%, and the structural unit D was contained in an amount of 2wt%, based on the total weight of said functional polymer.
The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.
The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.2

(1) 55.65g of monomer A', 12.25g of monomer B', 1.05g of monomer C' (M2) and 1.05g of monomer D' were taken, wherein the structure of said monomer A' was same as that in Example 1.1; the structure of said monomer B' was same as that in Example 1.1; the structure of said monomer D' was as shown in formula (d) (wherein $R_7$ was H and $R_8$ was methyl); the monomers were dissolved in 230g of deionized water, sodium hydroxide solution was used for adjusting the pH to 6, the starting temperature was controlled to 12°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate having a concentration of 0.2wt%, and 1.25g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.
(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.1);
Structural unit B (it was same as that in Example 1.1);
Structural unit C (its structure was as shown in formula (C), wherein $R_3$ was n-dodecyl, $R_4$ was H, X was O, $R_6$' was methyl, $R_5$, $R_7$, $R_6$ and $R_7$' were each H, n1 was 6, n2 was 9);
Structural unit D (its structure was as shown in formula (D), wherein $R_7$ was H and $R_8$ was methyl);
Wherein the structural unit A was contained in an amount of 79.5wt%, the structural unit B was contained in an amount of 17.5wt%, the structural unit C was contained in an amount of 1.5wt%, and the structural unit D was contained in an amount of 1.5wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.
The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.3

(1) 55.1g of monomer A', 13.7g of monomer B', 1.8g of monomer C' (M3) and 1.45g of monomer D' were taken, wherein said monomer A', said monomer B' and said monomer D' were same as those in Example 1.1; the monomers were dissolved in 228g of deionized water, sodium hydroxide solution was used for adjusting the pH to 6, the starting temperature was controlled to 15°C, nitrogen gas was blown into the system for 20min to remove oxygen gas, the system was then added with 1g of an aqueous solution of 2,2-azobis (2-amidinopropane) dihydrochloride having a concentration of 0.25wt%, 2g of an aqueous solution of ammonium persulfate having a concentration of 0.2wt%, and 1.5g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.1);
Structural unit B (it was same as that in Example 1.1);
Structural unit C (its structure was as shown in formula (C), wherein $R_3$ was 11-methyl dodecyl, $R_4$ was methyl, X was O, $R_5$' was methyl, $R_5$, $R_6$ and $R_6$' were each H, n1 was 2, n2 was 11);
Structural unit D (it was the same as that in Example 1.1);
Wherein the structural unit A was contained in an amount of 76.5wt%, the structural unit B was contained in an amount of 19wt%, the structural unit C was contained in an amount of 2.5wt%, and the structural unit D was contained in an amount of 2wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.

The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.4

(1) 41g of monomer A', 10.8g of monomer B', 3.7g of monomer C' (M4) and 4.5g of monomer D' were taken, wherein the structure of said monomer A' was represented by formula (a) (wherein $R_1$ was H);

The structure of said monomer B' was represented by formula (b-1) (wherein $R_2$' was H, M' was Na);
The structure of said monomer D' was represented by formula (d) (wherein $R_7$ and $R_8$ were each H);
The monomers were dissolved in 240g of deionized water, sodium hydroxide solution was used for adjusting the pH to 6, the starting temperature was controlled to 10°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate having a concentration of 0.2wt%, and 1.25g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (its structure was as shown in formula (A), wherein $R_1$ was H);

Structural unit B (its structure was as shown in formula (B-1), wherein $R_2$' was H, M' was Na);
Structural unit C (its structure was as shown in formula (C'), wherein $R_3$ was n-hexyl, $R_4$, $R_5$ and $R_6$ were each H, X was O, n was 0);
Structural unit D (its structure was as shown in formula (D), wherein $R_7$ and $R_8$ were each H);
Wherein the structural unit A was contained in an amount of 68.3wt%, the structural unit B was contained in an amount of 18wt%, the structural unit C was contained in an amount of 6.2wt%, and the structural unit D was contained in an amount of 7.5wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.
The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.5

(1) 46.2g of monomer A', 13.6g of monomer B', 5.3g of monomer C' (M5) and 4.9g of monomer D' were taken, wherein said monomer A' was same as that in Example 1.4, said monomer B' was same as that in Example 1.4, the structure of said monomer D' was represented by formula (d) (wherein $R_7$ was H, $R_8$ was methyl); the monomers were dissolved in 230g of deionized water, sodium hydroxide solution was used for adjusting the pH to 6, the starting temperature was controlled to 12°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate having a concentration of 0.2wt%, and 1.25g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.
(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.4);
Structural unit B (it was same as that in Example 1.4);
Structural unit C (its structure was as shown in formula (C'), wherein $R_3$ was n-dodecyl, $R_4$, $R_5$ and $R_6$ were each H, X was O, n was 0);
Structural unit D (its structure was as shown in formula (D), wherein $R_7$ was H, $R_8$ was methyl);
Wherein the structural unit A was contained in an amount of 66wt%, the structural unit B was contained in an amount of 19.4wt%, the structural unit C was contained in an amount of 7.6wt%, and the structural unit D was contained in an amount of 7wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.
The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.6

(1) 49.9g of monomer A', 12.6g of monomer B', 5g of monomer C' (M6) and 4.5g of monomer D' were taken, wherein said monomer A', said monomer B' and said monomer D' were same as those in Example 1.4; the monomers were dissolved in 228g of deionized water, sodium hydroxide solution was used for adjusting the pH to 6, the starting temperature was controlled to 15°C, nitrogen gas was blown into the system for 20min to remove oxygen gas, the system was then added with 1g of an aqueous solution of 2,2-azobis (2-amidinopropane) dihydrochloride having a concentration of 0.25wt%, 2g of an aqueous solution of ammonium persulfate having a concentration of 0.2wt%, and 1.5g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.4);
Structural unit B (it was same as that in Example 1.4);
Structural unit C (its structure was as shown in formula (C'), wherein $R_3$ was n-decyl, $R_4$ was methyl, $R_5$ and $R_6$ were each H, X was O, n was 0);
Structural unit D (it was same as that in Example 1.4);
Wherein the structural unit A was contained in an amount of 69.3wt%, the structural unit B was contained in an amount of 17.5wt%, the structural unit C was contained in an amount of 6.9wt%, and the structural unit D was contained in an amount of 6.3wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.

The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.7

(1) 46.8g of monomer A', 11g of monomer B', 3.6g of monomer C' (M7) and 3.6g of monomer D' were taken, wherein said monomer A', said monomer B' and said monomer D' were same as those in Example 1.4; the monomers were dissolved in 237g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.4);
Structural unit B (it was same as that in Example 1.4);
Structural unit C (its structure was as shown in formula (C'), wherein $R_3$ was straight chain C8 alkyl and straight chain C10 alkyl, $R_4$, $R_5$ and $R_6$ were each H, X was O, n was 8);
Structural unit D (it was same as that in Example 1.4);

Wherein the structural unit A was contained in an amount of 72wt%, the structural unit B was contained in an amount of 17wt%, the structural unit C was contained in an amount of 5.5wt%, and the structural unit D was contained in an amount of 5.5wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.

The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.8

(1) 49.4g of monomer A', 11.3g of monomer B', 4g of monomer C' (M8) and 3.3g of monomer D' were taken, wherein said monomer A', said monomer B' and said monomer D' were same as those in Example 1.4; the monomers were dissolved in 232g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.4);
Structural unit B (it was same as that in Example 1.4);
Structural unit C (its structure was as shown in formula (C'), wherein $R_3$ was straight chain C12 alkyl and straight chain C14 alkyl, $R_4$, $R_5$ and $R_6$ were each H, X was O, n was 4);
Structural unit D (it was same as that in Example 1.4);
Wherein the structural unit A was contained in an amount of 72.6wt%, the structural unit B was contained in an amount of 16.6wt%, the structural unit C was contained in an amount of 5.9wt%, and the structural unit D was contained in an amount of 4.9wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.

The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.9

(1) 63.3g of monomer A', 14.1g of monomer B', 3.8g of monomer C' (M9) and 3g of monomer D' were taken, wherein said monomer A', said monomer B' and said monomer D' were same as those in Example 1.4; the monomers were dissolved in 215.5g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was

continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.4);
Structural unit B (it was same as that in Example 1.4);
Structural unit C (its structure was as shown in formula (C'), wherein $R_3$ was straight chain C12 alkyl and straight chain C14 alkyl, $R_4$, $R_5$ and $R_6$ were each H, X was O, n was 7);
Structural unit D (it was same as that in Example 1.4);
Wherein the structural unit A was contained in an amount of 75.2wt%, the structural unit B was contained in an amount of 16.7wt%, the structural unit C was contained in an amount of 4.5wt%, and the structural unit D was contained in an amount of 3.6wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.

The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.10

(1) 44.4g of monomer A', 16.5g of monomer B', 7.1g of monomer C' and 7g of monomer D' were taken, wherein said monomer A', said monomer B', said monomer C' and said monomer D' were same as those in Example 1.4; the monomers were dissolved in 225g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.4);
Structural unit B (it was same as that in Example 1.4);
Structural unit C (it was same as that in Example 1.4);
Structural unit D (it was same as that in Example 1.4);
Wherein the structural unit A was contained in an amount of 59.2wt%, the structural unit B was contained in an amount of 22wt%, the structural unit C was contained in an amount of 9.5wt%, and the structural unit D was contained in an amount of 9.3wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.

The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.11

(1) 74.25g of monomer A', 13.5g of monomer B', 0.9g of monomer C' and 1.35g of monomer D' were taken, wherein said monomer A', said monomer B', said monomer C' and said monomer D' were same as those in Example 1.2; the monomers were dissolved in 234g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 1.2);
Structural unit B (it was same as that in Example 1.2);
Structural unit C (it was same as that in Example 1.2);
Structural unit D (it was same as that in Example 1.2);
Wherein the structural unit A was contained in an amount of 82.5wt%, the structural unit B was contained in an amount of 15wt%, the structural unit C was contained in an amount of 1wt%, and the structural unit D was contained in an amount of 1.5wt%, based on the total weight of said functional polymer.

The polymer solution with a concentration of 2,000mg/L was prepared by using the simulated brine having a mineralization degree of 30,000mg/L and a content of calcium and magnesium ions of 3,000mg/L, the apparent viscosity at 50°C was measured.

The polymer solution with a concentration of 1,000mg/L was prepared by using the simulated brine having a mineralization degree of 30,000mg/L and a content of calcium and magnesium ions of 3,000mg/L, the results of viscosity reduction rates of the heavy oil samples having a viscosity of 1,532mPa·s and 2,389mPa·s at 50°C respectively were illustrated in Table 2.

Example 1.11a

The polymer was prepared according to the same method as that in Example 1.11, except that the monomer C' was replaced by an equimolar amount of monomer (M5) in Preparation Example 1.5.

Example 1.11b

The polymer was prepared according to the same method as that in Example 1.11, except that the monomer C' was replaced by an equimolar amount of monomer (M7) in Preparation Example 1.7.

Example 1.11c

The polymer was prepared according to the same method as that in Example 1.11, except that the monomer C' used M11.

In addition, it was determined from the calculation based on the feeding amounts, the prepared functional polymer comprised:

Structural unit A (it was same as that in Example 1.11);
Structural unit B (it was same as that in Example 1.11);
Structural unit C (its structure was represented by formula (C), wherein $R_3$ was 2-ethylhexyl, $R_4$ was H, X was O, $R_5$ was methyl, $R_6$, $R_5$' and $R_6$' were each H, n1 was 3, n2 was 6);
Structural unit D (it was same as that in Example 1.11).

Comparative Example 1.1

The polymer was prepared according to the same method as that in Example 1.4, except that the monomer C' and monomer D' were not added.

It was determined from the calculation based on the feeding amounts, the prepared polymer comprised structural unit A and structural unit B which were identical with those of Example 1.4; the structural unit A was contained in an amount of 79.2wt%, and the structural unit B was contained in an amount of 20.8wt%, based on the total weight of said polymer.

Comparative Example 1.2

The polymer was prepared according to the same method as that in Example 1.11, except that the monomer D' was not added.

It was determined from the calculation based on the feeding amounts, the prepared polymer comprised structural unit A, structural unit B and structural unit C which were identical with those of Example 1.11; the structural unit A was contained in an amount of 83.8wt%, the structural unit B was contained in an amount of 15.2wt%, and the structural unit C was contained in an amount of 1wt%, based on the total weight of said polymer.

Comparative Example 1.3

The polymer was prepared according to the same method as that in Example 1.4, except that the monomer C' was not added.

It was determined from the calculation based on the feeding amounts, the prepared polymer comprised structural unit A, structural unit B and structural unit D which were identical with those of Example 1.4; the structural unit A was contained in an amount of 72.8wt%, the structural unit B was contained in an amount of 19.2wt%, and the structural unit D was contained in an amount of 8wt%, based on the total weight of said polymer.

Comparative Example 1.4

The polymer was prepared according to the same method as that in Example 1.11, except that the monomer C' was replaced by an equimolar amount of M10.

Comparative Example 1.5

The polymer was prepared according to the same method as that in Example 1.11, except that the monomer D' was replaced by an equimolar amount of vinyl pyrrolidone.

Table 2. Test results of viscosity average molecular weight, apparent viscosity and viscosity reduction rate

| No. | Viscosity average molecular weight (10,000) | Apparent viscosity (mPa·s) | Viscosity reduction rate (%) | |
|---|---|---|---|---|
| | | | Viscosity reduction rate of the heavy oil having a viscosity of 1,532mPa·s (50°C) | Viscosity reduction rate of the heavy oil having a viscosity of 2,389mPa·s (50°C) |
| Example 1.1 | 1420 | 76.5 | 97.6 | 98.8 |
| Example 1.2 | 1380 | 75.4 | 97.9 | 98.7 |
| Example 1.3 | 1350 | 70.5 | 97.8 | 98.9 |
| Example 1.4 | 1123 | 57.8 | 97.3 | 98.5 |
| Example 1.5 | 996 | 59.0 | 97.4 | 98.6 |
| Example 1.6 | 1054 | 60.5 | 96.6 | 98.1 |
| Example 1.7 | 1237 | 59.6 | 97.7 | 98.6 |
| Example 1.8 | 1316 | 64.1 | 96.5 | 98.3 |
| Example 1.9 | 1340 | 65.2 | 96.2 | 97.8 |
| Example 1.10 | 850 | 50.5 | 95.9 | 97.3 |
| Example 1.11 | 1450 | 78.0 | 92.0 | 93.5 |

(continued)

| No. | Viscosity average molecular weight (10,000) | Apparent viscosity (mPa·s) | Viscosity reduction rate (%) | |
|---|---|---|---|---|
| | | | Viscosity reduction rate of the heavy oil having a viscosity of 1,532mPa·s (50°C) | Viscosity reduction rate of the heavy oil having a viscosity of 2,389mPa·s (50°C) |
| Example 1.11a | 1435 | 77.2 | 77.3 | 78.1 |
| Example 1.11b | 1468 | 78.1 | 83.7 | 84.4 |
| Example 1.11c | 1400 | 75.8 | 89.1 | 90.6 |
| Comparative Example 1.1 | 1950 | 43.4 | 0 | 0 |
| Comparative Example 1.2 | 1475 | 78.4 | 73.1 | 74.5 |
| Comparative Example 1.3 | 1730 | 59.6 | 22.0 | 34.4 |
| Comparative Example 1.4 | 760 | 40.5 | 51.3 | 55.1 |
| Comparative Example 1.5 | 1010 | 46.5 | 67.1 | 68.2 |

Example 1.12

A rock core with a permeability of $900\times10^{-3}\mu m^2$ was selected, it was vacuumized for more than 2 hours and then saturated with simulated brine (with a mineralization degree of 30,000mg/L and a content of calcium and magnesium ions of 3,000mg/L), the rock core was saturated with a crude oil having a viscosity of 1,532mPa·s at 50°C, and the simulated brine was used for water flooding until that the water content at the rock core model outlet was over 98%, the water flooding recovery rate was 40.1%. The spent liquor and the dead volume were evacuated, 0.5PV of an aqueous solution of the polymer in Example 1.3 with a concentration of 1,000mg/L was injected, and the displacement was continued until that the water content reached 98% or more, the post-injection recovery rate was determined as 60.3%, the oil displacement efficiency was improved by 20.2%.

Example 1.13

A rock core with a permeability of $900\times10^{-3}\mu m^2$ was selected, it was vacuumized for more than 2 hours and then saturated with simulated brine (with a mineralization degree of 30,000mg/L and a content of calcium and magnesium ions of 3,000mg/L), the rock core was saturated with a crude oil having a viscosity of 1,532mPa·s at 50°C, and the simulated brine was used for water flooding until that the water content at the rock core model outlet was over 98%, the water flooding recovery rate was 39.8%. The spent liquor and the dead volume were evacuated, 0.5PV of an aqueous solution of the polymer in Example 1.4 with a concentration of 1,000mg/L was injected, and the displacement was continued until that the water content reached 98% or more, the post-injection recovery rate was determined as 56.5%, the oil displacement efficiency was improved by 16.7%.

Example 1.14

A rock core with a permeability of $1,000\times10^{-3}\mu m^2$ was selected, it was vacuumized for more than 2 hours and then saturated with simulated brine (with a mineralization degree of 30,000mg/L and a content of calcium and magnesium ions of 3,000mg/L), the rock core was saturated with a crude oil having a viscosity of 2,389mPa·s at 50°C, and the simulated brine was used for water flooding until that the water content at the rock core model outlet was over 98%, the water flooding recovery rate was 20.07%. The spent liquor and the dead volume were evacuated, 0.5PV of an aqueous solution of the polymer in Example 1.7 with a concentration of 1,000mg/L was injected, and the displacement was continued until that the water content reached 98% or more, the post-injection recovery rate was determined as 37.27%, the oil displacement efficiency was improved by 17.2 %.

Example 1.15

A rock core with a permeability of $1,000\times10^{-3}\mu m^2$ was selected, it was vacuumized for more than 2 hours and then saturated with simulated brine (with a mineralization degree of 30,000mg/L and a content of calcium and magnesium ions of 3,000mg/L), the rock core was saturated with a crude oil having a viscosity of 2,389mPa·s at 50°C, and the

simulated brine was used for water flooding until that the water content at the rock core model outlet was over 98%, the water flooding recovery rate was 19.48%. The spent liquor and the dead volume were evacuated, 0.5PV of an aqueous solution of the polymer in Example 1.11 with a concentration of 1,000mg/L was injected, and the displacement was continued until that the water content reached 98% or more, the post-injection recovery rate was determined as 33.4%, the oil displacement efficiency was improved by 14.22%.

Comparative Example 1.6

A rock core with a permeability of $1,000\times10^{-3}\mu m^2$ was selected, it was vacuumized for more than 2 hours and then saturated with simulated brine (with a mineralization degree of 30,000mg/L and a content of calcium and magnesium ions of 3,000mg/L), the rock core was saturated with a crude oil having a viscosity of 2,389mPa·s at 50°C, and the simulated brine was used for water flooding until that the water content at the rock core model outlet was over 98%, the water flooding recovery rate was 34.51%. The spent liquor and the dead volume were evacuated, 0.5PV of an aqueous solution of the polymer in Comparative Example 1.1 with a concentration of 1,000mg/L was injected, and the displacement was continued until that the water content reached 98% or more, the post-injection recovery rate was determined as 41.68%, the oil displacement efficiency was improved by 7.17 %.

Comparative Example 1.7

A rock core with a permeability of $1,000\times10^{-3}\mu m^2$ was selected, it was vacuumized for more than 2 hours and then saturated with simulated brine (with a mineralization degree of 30,000mg/L and a content of calcium and magnesium ions of 3,000mg/L), the rock core was saturated with a crude oil having a viscosity of 2,389mPa·s at 50°C, and the simulated brine was used for water flooding until that the water content at the rock core model outlet was over 98%, the water flooding recovery rate was 32.41%. The spent liquor and the dead volume were evacuated, 0.5PV of an aqueous solution of the polymer in Comparative Example 1.2 with a concentration of 1,000mg/L was injected, and the displacement was continued until that the water content reached 98% or more, the post-injection recovery rate was determined as 42.4%, the oil displacement efficiency was improved by 9.99%.

Comparative Example 1.8

A rock core with a permeability of $1,000\times10^{-3}\mu m^2$ was selected, it was vacuumized for more than 2 hours and then saturated with simulated brine (with a mineralization degree of 30,000mg/L and a content of calcium and magnesium ions of 3,000mg/L), the rock core was saturated with a crude oil having a viscosity of 2,389mPa·s at 50°C, and the simulated brine was used for water flooding until that the water content at the rock core model outlet was over 98%, the water flooding recovery rate was 30.45%. The spent liquor and the dead volume were evacuated, 0.5PV of an aqueous solution of the polymer in Comparative Example 1.3 with a concentration of 1,000mg/L was injected, and the displacement was continued until that the water content reached 98% or more, the post-injection recovery rate was determined as 38.82%, the oil displacement efficiency was improved by 8.37%.

Although not shown, the polymers obtained from the other examples were also able to improve the oil displacement efficiency by 14-21% under the same conditions, while the polymers prepared in Comparative Examples 1.1 to 1.3 had an improvement less than 10.5% in the oil displacement efficiency.

As illustrated by the above results, in comparison with Comparative Examples, the functional polymer prepared from the compound of the present invention has desirable water phase thickening function at 50°C in water having a mineralization degree of 30,000mg/L and a content of calcium and magnesium ions of 3,000mg/L, in addition, under the conditions of a low driving force and a concentration of 1,000mg/L, the viscosity of the heavy oil having a viscosity of 1,500-3,000mPa·s under the oil reservoir temperature can be reduced by more than 92%, and the water-flooding recovery rate of the heavy oil reservoir can be significantly improved.

Example 2.1

(1) 43.9g of monomer A', 11.14g of monomer B', 0.88g of monomer C' (N1) and 1.12g of monomer D' were taken, wherein the structure of said monomer A' was as shown in formula (a) (wherein $R_1$ was H);

The structure of said monomer B' was as shown in formula (b-2) (wherein $R_2''$ was H, $R_2$ was $SO_3M$, M=Na);
The structure of said monomer D' was as shown in formula (d) (wherein $R_7$ and $R_8$ were each H);
The monomers were dissolved in 243g of deionized water, sodium hydroxide solution was used for adjusting the

pH to 6, the starting temperature was controlled to 10°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate having a concentration of 0.2wt%, and 1.25g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (its structure was as shown in formula (A), wherein $R_1$ was H);
Structural unit B (its structure was as shown in formula (B-2), wherein $R_2$" was H, $R_2$ was $SO_3M$, M = Na);
Structural unit C (its structure was as shown in formula (C), wherein $R_3$ was 2-ethylhexyl, $R_4$ was H, X was NH, $R_5$' was methyl, $R_5$, $R_6$ and $R_6$' were each H, n1 was 3, n2 was 6);
Structural unit D (its structure was as shown in formula (D), wherein $R_7$ and $R_8$ were each H);
Wherein the structural unit A was contained in an amount of 77wt%, the structural unit B was contained in an amount of 19.5wt%, the structural unit C was contained in an amount of 1.5wt%, and the structural unit D was contained in an amount of 2wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 3.

Example 2.2

(1) 55g of monomer A', 12.6g of monomer B', 1.4g of monomer C' (N2) and 1.05g of monomer D' were taken, wherein the structure of said monomer A' was same as that in Example 2.1; the structure of said monomer B' was same as that in Example 2.1; the structure of said monomer D' was as shown in formula (d) (wherein $R_7$ was H, $R_8$ was methyl); the monomers were dissolved in 230g of deionized water, sodium hydroxide solution was used for adjusting the pH to 6, the starting temperature was controlled to 12°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate having a concentration of 0.2wt%, and 1.8g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.
(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.1);
Structural unit B (it was same as that in Example 2.1);
Structural unit C (its structure was as shown in formula (C), wherein $R_3$ was n-dodecyl, X was NH, $R_5$ was methyl, $R_5$, $R_6$ and $R_6$' were each H, n1 was 6, n2 was 9);
Structural unit D (its structure was as shown in formula (D), wherein $R_7$ was H, $R_8$ was methyl);
Wherein the structural unit A was contained in an amount of 78.5wt%, the structural unit B was contained in an amount

of 18wt%, the structural unit C was contained in an amount of 2wt%, and the structural unit D was contained in an amount of 1.5wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 3.

Example 2.3

(1) 56.6g of monomer A', 15g of monomer B', 1.9g of monomer C' (N3) and 1.5g of monomer D' were taken, wherein said monomer A', said monomer B' and said monomer D' were same as those in Example 2.1; the monomers were dissolved in 225g of deionized water, sodium hydroxide solution was used for adjusting the pH to 6, the starting temperature was controlled to 15°C, nitrogen gas was blown into the system for 20min to remove oxygen gas, the system was then added with 1g of an aqueous solution of 2,2-azobis (2-amidinopropane) dihydrochloride having a concentration of 0.25wt%, 2g of an aqueous solution of ammonium persulfate having a concentration of 0.2wt%, and 1.7g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.1);
Structural unit B (it was same as that in Example 2.1);
Structural unit C (its structure was as shown in formula (C), wherein $R_3$ was 11-methyl dodecyl, $R_4$ was methyl, X was NH, $R_5$' was methyl, $R_5$, $R_6$ and $R_6$' were each H, n1 was 2, n2 was 11);
Structural unit D (it was the same as that in Example 2.1);
Wherein the structural unit A was contained in an amount of 75.5wt%, the structural unit B was contained in an amount of 20wt%, the structural unit C was contained in an amount of 2.5wt%, and the structural unit D was contained in an amount of 2wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 2.

Example 2.4

(1) 41.4g of monomer A', 9.5g of monomer B', 4.1g of monomer C' (N4) and 2g of monomer D' were taken, wherein the structure of said monomer A' was represented by formula (a) (wherein $R_1$ was H);

The structure of said monomer B' was represented by formula (b-2) (wherein $R_2$" was H, $R_2$ was $SO_3M$, M=H);
The structure of said monomer D' was represented by formula (d) (wherein $R_7$ and $R_8$ were each H);
The monomers were dissolved in 243g of deionized water, sodium hydroxide solution was used for adjusting the pH to 6, the starting temperature was controlled to 10°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate

having a concentration of 0.2wt%, and 1.25g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (its structure was as shown in formula (A), wherein $R_1$ was H);
Structural unit B (its structure was as shown in formula (B-2), wherein $R_2''$ was H, $R_2$ was $SO_3M$, M=H);
Structural unit C (its structure was as shown in formula (C'), wherein $R_3$ was n-hexyl, $R_4$, $R_5$ and $R_6$ were each H, X was NH, n was 0);
Structural unit D (its structure was as shown in formula (D), wherein $R_7$ and $R_8$ were each H);
Wherein the structural unit A was contained in an amount of 72.6wt%, the structural unit B was contained in an amount of 16.7wt%, the structural unit C was contained in an amount of 7.2wt%, and the structural unit D was contained in an amount of 3.5wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 3.

Example 2.5

(1) 52.5g of monomer A', 10.8g of monomer B', 4.6g of monomer C' (N5) and 2.1g of monomer D' were taken, wherein said monomer A' was same as that in Example 1, said monomer B' was same as that in Example 1, the structure of said monomer D' was represented by formula (d) (wherein $R_7$ was H, $R_8$ was methyl); the monomers were dissolved in 230g of deionized water, sodium hydroxide solution was used for adjusting the pH to 6, the starting temperature was controlled to 12°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate having a concentration of 0.2wt%, and 1.8g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.
(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.4);
Structural unit B (it was same as that in Example 2.4);
Structural unit C (its structure was as shown in formula (C'), wherein $R_3$ was n-dodecyl, $R_4$, $R_5$ and $R_6$ were each H, X was NH, n was 0);
Structural unit D (its structure was as shown in formula (D), wherein $R_7$ was H, $R_8$ was methyl);
Wherein the structural unit A was contained in an amount of 75wt%, the structural unit B was contained in an amount of 15.4wt%, the structural unit C was contained in an amount of 6.6wt%, and the structural unit D was contained in an amount of 3wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 3.

Example 2.6

(1) 56.2g of monomer A', 13.6g of monomer B', 3.5g of monomer C' (N6) and 1.73g of monomer D' were taken, wherein said monomer A', said monomer B' and said monomer D' were same as those in Example 2.4; the monomers were dissolved in 225g of deionized water, sodium hydroxide solution was used for adjusting the pH to 6, the starting temperature was controlled to 15°C, nitrogen gas was blown into the system for 20min to remove oxygen gas, the system was then added with 1g of an aqueous solution of 2,2-azobis (2-amidinopropane) dihydrochloride having a concentration of 0.25wt%, 2g of an aqueous solution of ammonium persulfate having a concentration of 0.2wt%, and 1.7g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.4);
Structural unit B (it was same as that in Example 2.4);
Structural unit C (its structure was as shown in formula (C'), wherein $R_3$ was n-decyl, $R_4$ was methyl, $R_5$ and $R_6$ were each H, X was NH, n was 0);
Structural unit D (it was same as that in Example 2.4);
Wherein the structural unit A was contained in an amount of 74.9wt%, the structural unit B was contained in an amount of 18.1wt%, the structural unit C was contained in an amount of 4.7wt%, and the structural unit D was contained in an amount of 2.3wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 2.

Example 2.7

(1) 45.1g of monomer A', 12.3g of monomer B', 3.2g of monomer C' (N7) and 2.4g of monomer D' were taken, wherein said monomer A' and said monomer D' were same as those in Example 2.4, the structure of said monomer B' was represented by formula (b-2) (wherein $R_2''$ was H, $R_2$ was $P(O)(OM)_2$, M=H); the monomers were dissolved in 237g of deionized water, sodium hydroxide solution was used for adjusting the pH to 7, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.4);
Structural unit B (its structure was represented by formula (B-2), wherein $R_2$" was H, $R_2$ was $P(O)(OM)_2$, M=Na);
Structural unit C (its structure was as shown in formula (C'), wherein $R_3$ was straight chain C8 alkyl and straight chain C10 alkyl, $R_4$, $R_5$ and $R_6$ were each H, X was NH, n was 10);
Structural unit D (it was same as that in Example 2.4);
Wherein the structural unit A was contained in an amount of 71.6wt%, the structural unit B was contained in an amount of 19.5wt%, the structural unit C was contained in an amount of 5.1wt%, and the structural unit D was contained in an amount of 3.8wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 3.

Example 2.8

(1) 48.7g of monomer A', 11.6g of monomer B', 3.9g of monomer C' (N8) and 1.8g of monomer D' were taken, wherein said monomer A', said monomer B' and said monomer D' were same as those in Example 2.4; the monomers were dissolved in 240g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.
(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.4);
Structural unit B (it was same as that in Example 2.4);
Structural unit C (its structure was as shown in formula (C'), wherein $R_3$ was straight chain C12 alkyl and straight chain C14 alkyl, $R_4$, $R_5$, $R_6$ and $R_7$ were each H, X was NH, n was 4);
Structural unit D (it was same as that in Example 2.4);
Wherein the structural unit A was contained in an amount of 73.8wt%, the structural unit B was contained in an amount of 17.6wt%, the structural unit C was contained in an amount of 5.9wt%, and the structural unit D was contained in an amount of 2.7wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity

reduction rates of the heavy oil samples having a viscosity of 1,361mPa·s and 2,630mPa·s at 50°C respectively were illustrated in Table 3.

Example 2.9

(1) 63.3g of monomer A', 16.8g of monomer B', 7g of monomer C' (N9) and 2.9g of monomer D' were taken, wherein said monomer A', said monomer B' and said monomer D' were same as those in Example 2.4; the monomers were dissolved in 234g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 2g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, a functional polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.4);
Structural unit B (it was same as that in Example 2.4);
Structural unit C (its structure was as shown in formula (C'), wherein $R_3$ was straight chain C12 alkyl and straight chain C14 alkyl, $R_4$, $R_5$ and $R_6$ were each H, X was NH, n was 7);
Structural unit D (it was same as that in Example 2.4);
Wherein the structural unit A was contained in an amount of 70.3wt%, the structural unit B was contained in an amount of 18.7wt%, the structural unit C was contained in an amount of 7.8wt%, and the structural unit D was contained in an amount of 3.2wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 3.

Example 2.10

(1) 44.3g of monomer A', 12g of monomer B', 2.5g of monomer C' (N10) and 1.2g of monomer D' were taken, wherein said monomer A', said monomer B' and said monomer D' were same as those in Example 2.4; the monomers were dissolved in 220g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional

polymer comprised:

Structural unit A (it was same as that in Example 2.4);
Structural unit B (it was same as that in Example 2.4);
Structural unit C (its structure was as shown in formula (C'), wherein $R_3$ was straight chain C8 alkyl and straight chain C10 alkyl, $R_4$, $R_5$ and $R_6$ were each H, X was NH, n was 5);
Structural unit D (it was same as that in Example 2.4);
Wherein the structural unit A was contained in an amount of 73.8wt%, the structural unit B was contained in an amount of 20wt%, the structural unit C was contained in an amount of 4.2wt%, and the structural unit D was contained in an amount of 2wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.
The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 3.

Example 2.11

(1) 43.6g of monomer A', 15.5g of monomer B', 5.9g of monomer C' and 1g of monomer D' were taken, wherein said monomer A', said monomer B', said monomer C' and said monomer D' were same as those in Example 2.4; the monomers were dissolved in 234g of deionized water, sodium hydroxide solution was used for adjusting the pH to 6, the starting temperature was controlled to 15°C, nitrogen gas was blown into the system for 20min to remove oxygen gas, the system was then added with 1.1g of an aqueous solution of 2,2-azobis (2-amidinopropane) dihydrochloride having a concentration of 0.25wt%, 2g of an aqueous solution of ammonium persulfate having a concentration of 0.2wt%, and 1.6g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.
(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.4);
Structural unit B (it was same as that in Example 2.4);
Structural unit C (it was same as that in Example 2.4);
Structural unit D (it was same as that in Example 2.4);
Wherein the structural unit A was contained in an amount of 66.1wt%, the structural unit B was contained in an amount of 23.5wt%, the structural unit C was contained in an amount of 8.9wt%, and the structural unit D was contained in an amount of 1.5wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.
The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 2.

Example 2.12

(1) 71.55g of monomer A', 16.2g of monomer B', 0.9g of monomer C' and 1.35g of monomer D' were taken, wherein said monomer A', said monomer B', said monomer C' and said monomer D' were same as those in Example 2.1; the monomers were dissolved in 210g of deionized water, sodium hydroxide solution was used for adjusting the pH to 6, the starting temperature was controlled to 15°C, nitrogen gas was blown into the system for 20min to remove oxygen gas, the system was then added with 1.15g of an aqueous solution of 2,2-azobis (2-amidinopropane) dihydrochloride having a concentration of 0.25wt%, 2g of an aqueous solution of ammonium persulfate having a concentration of 0.2wt%, and 2.2g of an aqueous solution of sodium bisulfite having a concentration of 0.3wt% to initiate the polymerization, after the system temperature was raised by 0.5°C, the blowing of nitrogen gas bubbles was stopped, and the reaction was continued for 4h.

(2) After completion of the polymerization process, the resulting colloid was granulated, dried at a temperature of 50°C to a solid content of 89wt%, then crushed and sieved, an acrylamide polymer dry powder product was obtained. The viscosity average molecular weight of the dry powder product was measured.

In addition, it was determined from the calculation based on the feeding amounts, the prepared functional polymer comprised:

Structural unit A (it was same as that in Example 2.1);
Structural unit B (it was same as that in Example 2.1);
Structural unit C (it was same as that in Example 2.1);
Structural unit D (it was same as that in Example 2.1);
Wherein the structural unit A was contained in an amount of 79.5wt%, the structural unit B was contained in an amount of 18wt%, the structural unit C was contained in an amount of 1wt%, and the structural unit D was contained in an amount of 1.5wt%, based on the total weight of said functional polymer.

The polymer solution with a concentration of 2,000mg/L was prepared by using the simulated brine having a mineralization degree of 50,000mg/L and a content of calcium and magnesium ions of 5,000mg/L, the apparent viscosity at 50°C was measured.

The polymer solution with a concentration of 1,000mg/L was prepared by using the simulated brine having a mineralization degree of 50,000mg/L and a content of calcium and magnesium ions of 5,000mg/L, the results of viscosity reduction rates of the heavy oil samples having a viscosity of 1,361mPa·s and 2,630mPa·s at 50°C respectively were illustrated in Table 3.

Example 2.12a

The polymer was prepared according to the same method as that in Example 2.12, except that the monomer C' was replaced by an equimolar amount of monomer in Preparation Example 2.5.

Example 2.12b

The polymer was prepared according to the same method as that in Example 2.12, except that the monomer C' was replaced by an equimolar amount of monomer (N7) in Preparation Example 2.7.

Example 2.12c

The polymer was prepared according to the same method as that in Example 2.12, except that the monomer C' used N11.

Comparative Example 2.1

The polymer was prepared according to the same method as that in Example 2.4, except that the monomer C' and monomer D' were not added.

It was determined from the calculation based on the feeding amounts, the prepared polymer comprised structural unit A and structural unit B which were identical with those of Example 2.4; the structural unit A was contained in an amount of 81.3wt%, and the structural unit B was contained in an amount of 18.7wt%, based on the total weight of said polymer.

Comparative Example 2.2

The polymer was prepared according to the same method as that in Example 2.12, except that the monomer D'

was not added.

It was determined from the calculation based on the feeding amounts, the prepared polymer comprised structural unit A, structural unit B and structural unit C which were identical with those of Example 2.12; the structural unit A was contained in an amount of 80.7wt%, the structural unit B was contained in an amount of 18.3wt%, and the structural unit C was contained in an amount of 1wt%, based on the total weight of said polymer.

Comparative Example 2.3

The polymer was prepared according to the same method as that in Example 2.4, except that the monomer C' was not added.

It was determined from the calculation based on the feeding amounts, the prepared polymer comprised structural unit A, structural unit B and structural unit D which were identical with those of Example 2.4; the structural unit A was contained in an amount of 78.3wt%, the structural unit B was contained in an amount of 18wt%, and the structural unit D was contained in an amount of 3.7wt%, based on the total weight of said polymer.

Comparative Example 2.4

The polymer was prepared according to the same method as that in Example 2.12, except that the monomer C' was replaced by an equimolar amount of M10.

Comparative Example 2.5

The polymer was prepared according to the same method as that in Example 2.12, except that the monomer D' was replaced by an equimolar amount of vinyl pyrrolidone.

Table 3. Test results of viscosity average molecular weight, apparent viscosity and viscosity reduction rate

| No. | Viscosity average molecular weight (10,000) | Apparent viscosity (mPa·s) | Viscosity reduction rate (%) | |
|---|---|---|---|---|
| | | | Viscosity reduction rate of the heavy oil having a viscosity of 1,361mPa·s (50°C) | Viscosity reduction rate of the heavy oil having a viscosity of 2,630mPa·s (50°C) |
| Example 2.1 | 1430 | 77.4 | 97.4 | 98.6 |
| Example 2.2 | 1405 | 76.5 | 97.8 | 98.8 |
| Example 2.3 | 1365 | 71.6 | 97.6 | 98.7 |
| Example 2.4 | 1150 | 50.4 | 97.0 | 98.2 |
| Example 2.5 | 1035 | 47.8 | 97.2 | 98.3 |
| Example 2.6 | 1124 | 53.1 | 96.8 | 98.1 |
| Example 2.7 | 985 | 42.8 | 97.5 | 98.5 |
| Example 2.8 | 1176 | 52.3 | 97.6 | 98.7 |
| Example 2.9 | 956 | 39.2 | 96.1 | 97.9 |
| Example 2.10 | 1189 | 45.0 | 95.9 | 97.8 |
| Example 2.11 | 832 | 35.5 | 95.5 | 97.3 |
| Example 2.12 | 1440 | 78.6 | 91.8 | 93.3 |
| Example 2.12a | 1285 | 50.7 | 73.1 | 75.2 |
| Example 2.12b | 1475 | 78.9 | 80.3 | 82.2 |
| Example 2.12c | 1418 | 76.7 | 87.3 | 88.9 |
| Comparative Example 2.1 | 1980 | 30.5 | 0 | 0 |
| Comparative Example 2.2 | 1455 | 76.4 | 69.2 | 70.7 |
| Comparative Example 2.3 | 1790 | 54.6 | 21.2 | 32.8 |

(continued)

| No. | Viscosity average molecular weight (10,000) | Apparent viscosity (mPa·s) | Viscosity reduction rate (%) | |
|---|---|---|---|---|
| | | | Viscosity reduction rate of the heavy oil having a viscosity of 1,361mPa·s (50°C) | Viscosity reduction rate of the heavy oil having a viscosity of 2,630mPa·s (50°C) |
| Comparative Example 2.4 | 685 | 28.5 | 46.3 | 47.2 |
| Comparative Example 2.5 | 836 | 32.4 | 66.5 | 67.6 |

Example 2.13

A rock core with a permeability of $800\times10^{-3}\mu m^2$ was selected, it was vacuumized for more than 2 hours and then saturated with simulated brine (with a mineralization degree of 50,000mg/L and a content of calcium and magnesium ions of 5,000mg/L), the rock core was saturated with a crude oil having a viscosity of 1,361mPa·s at 50°C, and the simulated brine was used for water flooding until that the water content at the rock core model outlet was over 98%, the water flooding recovery rate was 35.76%. The spent liquor and the dead volume were evacuated, 0.5PV of an aqueous solution of the polymer in Example 2.5 with a concentration of 1,000mg/L was injected, and the displacement was continued until that the water content reached 98% or more, the post-injection recovery rate was determined as 53.75%, the oil displacement efficiency was improved by 17.99%.

Example 2.14

A rock core with a permeability of $1,000\times10^{-3}\mu m^2$ was selected, it was vacuumized for more than 2 hours and then saturated with simulated brine (with a mineralization degree of 50,000mg/L and a content of calcium and magnesium ions of 5,000mg/L), the rock core was saturated with a crude oil having a viscosity of 2,630mPa·s at 50°C, and the simulated brine was used for water flooding until that the water content at the rock core model outlet was over 98%, the water flooding recovery rate was 36.77%. The spent liquor and the dead volume were evacuated, 0.5PV of an aqueous solution of the polymer in Example 2.8 with a concentration of 1,000mg/L was injected, and the displacement was continued until that the water content reached 98% or more, the post-injection recovery rate was determined as 56.39%, the oil displacement efficiency was improved by 19.62%.

Example 2.15

A rock core with a permeability of $860\times10^{-3}\mu m^2$ was selected, it was vacuumized for more than 2 hours and then saturated with simulated brine (with a mineralization degree of 50,000mg/L and a content of calcium and magnesium ions of 5,000mg/L), the rock core was saturated with a crude oil having a viscosity of 1,361mPa·s at 50°C, and the simulated brine was used for water flooding until that the water content at the rock core model outlet was over 98%, the water flooding recovery rate was 33.28%. The spent liquor and the dead volume were evacuated, 0.5PV of an aqueous solution of the polymer in Example 2.10 with a concentration of 1,000mg/L was injected, and the displacement was continued until that the water content reached 98% or more, the post-injection recovery rate was determined as 48.59%, the oil displacement efficiency was improved by 15.31%.

Comparative Example 2.6

A rock core with a permeability of $860\times10^{-3}\mu m^2$ was selected, it was vacuumized for more than 2 hours and then saturated with simulated brine (with a mineralization degree of 50,000mg/L and a content of calcium and magnesium ions of 5,000mg/L), the rock core was saturated with a crude oil having a viscosity of 1,361mPa·s at 50°C, and the simulated brine was used for water flooding until that the water content at the rock core model outlet was over 98%, the water flooding recovery rate was 30.64%. The spent liquor and the dead volume were evacuated, 0.5PV of an aqueous solution of the polymer in Comparative Example 2.1 with a concentration of 1,000mg/L was injected, and the displacement was continued until that the water content reached 98% or more, the post-injection recovery rate was determined as 37.85%, the oil displacement efficiency was improved by 7.21%.

Comparative Example 2.7

A rock core with a permeability of $860\times10^{-3}\mu m^2$ was selected, it was vacuumized for more than 2 hours and then

saturated with simulated brine (with a mineralization degree of 50,000mg/L and a content of calcium and magnesium ions of 5,000mg/L), the rock core was saturated with a crude oil having a viscosity of 1,361mPa·s at 50°C, and the simulated brine was used for water flooding until that the water content at the rock core model outlet was over 98%, the water flooding recovery rate was 31.29%. The spent liquor and the dead volume were evacuated, 0.5PV of an aqueous solution of the polymer in Comparative Example 2.2 with a concentration of 1,000mg/L was injected, and the displacement was continued until that the water content reached 98% or more, the post-injection recovery rate was determined as 36.15%, the oil displacement efficiency was improved by 9.23%.

Comparative Example 2.8

A rock core with a permeability of $860\times10^{-3}\mu m^2$ was selected, it was vacuumized for more than 2 hours and then saturated with simulated brine (with a mineralization degree of 50,000mg/L and a content of calcium and magnesium ions of 5,000mg/L), the rock core was saturated with a crude oil having a viscosity of 1,361mPa·s at 50°C, and the simulated brine was used for water flooding until that the water content at the rock core model outlet was over 98%, the water flooding recovery rate was 32.53%. The spent liquor and the dead volume were evacuated, 0.5PV of an aqueous solution of the polymer in Comparative Example 2.3 with a concentration of 1,000mg/L was injected, and the displacement was continued until that the water content reached 98% or more, the post-injection recovery rate was determined as 41.12%, the oil displacement efficiency was improved by 8.59%.

Although not shown, the polymers obtained from the other examples were also able to improve the oil displacement efficiency by 15-20% under the same conditions, while the polymers prepared in Comparative Examples 2.1 to 2.3 had an improvement less than 10% in the oil displacement efficiency.

As illustrated by the above results, in comparison with Comparative Examples, the functional polymer for cold extraction prepared from the compound of the present invention has desirable water phase thickening function at 50°C in water having a mineralization degree less than or equal to 50,000mg/L and a content of calcium and magnesium ions less than or equal to 5,000mg/L, in addition, under the conditions of a low driving force and a concentration of 1,000mg/L, the viscosity of the heavy oil having a viscosity of 1,500-3,000mPa·s under the oil reservoir temperature can be reduced by more than 91%, and the water-flooding recovery rate of the heavy oil reservoir can be significantly improved.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

## Claims

1. A glycoside group-containing compound, is **characterized in that** the compound has a structure represented by formula (1):

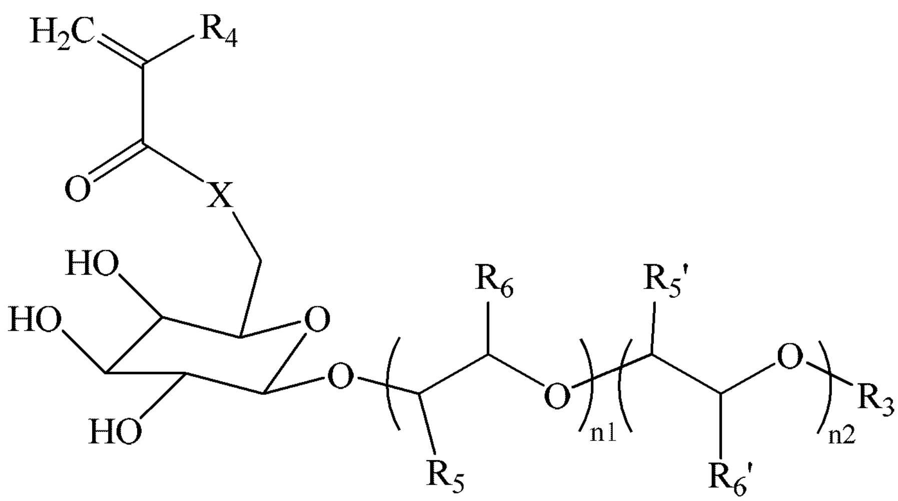

Formula (1)

in formula (1), $R_3$ is C4-C20 alkyl or C7-C20 alkylphenyl, $R_4$, $R_5$, $R_6$, $R_5$' and $R_6$' are each independently H or C1-C4

alkyl, X is O or NQ, Q is H or C1-C4 alkyl, the sum of n1 and n2 is within the range of 0-20, and when X is O, n1+ n2≠0.

**2.** The compound according to claim 1, wherein $R_3$ is C6-C18 alkyl;

and/or $R_4$, $R_5$, $R_6$, $R_5$' and $R_6$' are each independently H or methyl;
and/or n1+n2≠0.

**3.** The compound according to claim 1 or 2, wherein $R_3$ is n-hexyl, n-dodecyl, n-decyl, n-octyl, n-tetradecyl, 2-ethylhexyl or 11-methyldodecyl;

and/or $R_5$' and $R_6$' are each independently H or methyl and $R_5$' and $R_6$' are different, both $R_5$ and $R_6$ are H;
and/or each of n1 and n2 is independently 1, 2, 3, 4, 5, 6, 7, 8, 9 or 10.

**4.** A glycoside group-containing polymer, is **characterized in that** the polymer comprises a structural unit C represented by formula (C) and a structural unit D represented by formula (D):

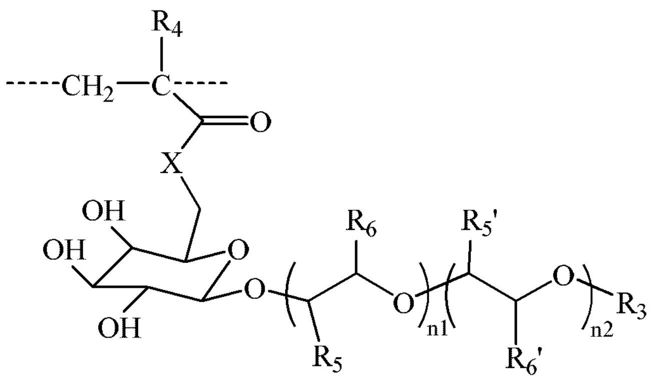
Formula(C),

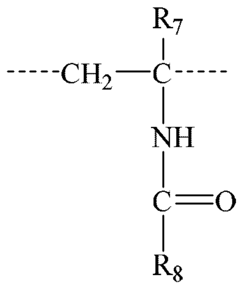
Formula(D)

wherein $R_3$ is C4-C20 alkyl or C7-C20 alkylphenyl, $R_4$, $R_5$, $R_6$, $R_5$' and $R_6$', $R_7$ and $R_8$ are each independently H or C1-C4 alkyl, X is O or NQ, Q is H or C1-C4 alkyl, n1+ n2=0-20.

**5.** The polymer according to claim 4, wherein the weight ratio of said structural unit C to said structural unit D is (5-300): (5-300);

and/or $R_3$ is C6-C18 alkyl;
and/or $R_4$, $R_5$, $R_6$, $R_5$', $R_6$', $R_7$, $R_8$ are each independently H or methyl;
and/or when X is O, n1+ n2≠0.

**6.** The polymer according to claim 4 or 5, wherein the weight ratio of said structural unit C to said structural unit D is (6-150): (8-200);

and/or $R_3$ is n-hexyl, n-dodecyl, n-decyl, n-octyl, n-tetradecyl, 2-ethylhexyl or 11-methyldodecyl; and/or $R_5$' and $R_6$' are each independently H or methyl and $R_5$' and $R_6$' are different, both $R_5$ and $R_6$ are H;
and/or nl+n2≠0; preferably, n1 and n2 are each independently 1, 2, 3, 4, 5, 6, 7, 8, 9 or 10.

**7.** The polymer according to any one of claims 4-6, wherein the polymer further comprises a structural unit represented by formula (C-1):

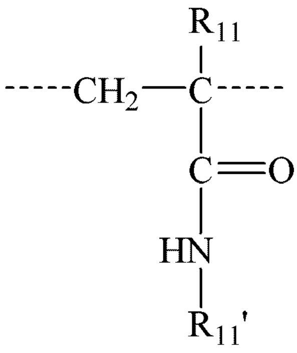
Formula (C-1)

wherein $R_{11}$ is H or C1-C4 alkyl; $R_{11}$' is H, C1-C4 alkyl, $SO_3M$-substituted C2-C6 alkyl, $P(O)(OM)_2$-substituted C2-C6 alkyl, M is H or an alkali metal element;
the weight ratio of said structural unit represented by formula (C-1) to said structural unit C is (1-1.8): (0.005-0.3), preferably (1-1.7): (0.006-0.15).

**8.** The polymer according to any one of claims 4-7, wherein the polymer comprises a structural unit A represented by formula (A), a structural unit B represented by formula (B-1) and/or (B-2), a structural unit C represented by formula (C), and a structural unit D represented by formula (D);

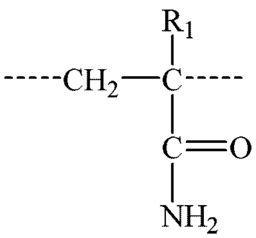
Formula (A),
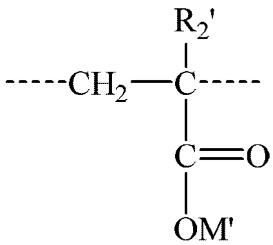
Formula (B-l),

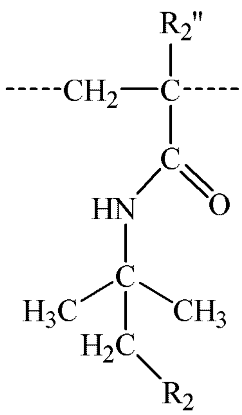
Formula (B-2),
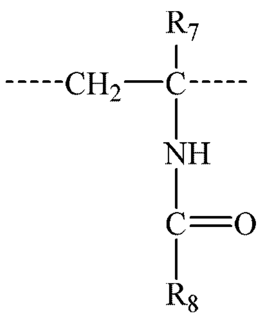
Formula (D);

wherein $R_1$, $R_2$', $R_2$" are each independently H or C1-C4 alkyl, $R_2$ is $SO_3M$ or $P(O)(OM)_2$, M and M' are each independently H or an alkali metal element.

**9.** The polymer according to claim 8, wherein $R_1$, $R_2$', $R_2$" are each independently H or methyl;

and/or M and M' are each independently H or Na;
and/or the weight ratio of said structural unit A, said structural unit B, said structural unit C and said structural unit D is 1: (0.02-0.8): (0.005-0.3): (0.005-0.3), preferably 1: (0.03-0.7): (0.006-0.15): (0.008-0.2).

**10.** The polymer according to any one of claims 4-9, wherein the polymer has a viscosity average molecular weight within the range of 6,000,000-18,000,000.

11. The polymer according to any one of claims 4-9, wherein the polymer has a viscosity average molecular weight within the range of 7,000,000-15,000,000.

12. A method for preparing a glycoside group-containing polymer, is **characterized in that** the method comprises: subjecting alkenyl monomers in an alkenyl monomer solution to a polymerization reaction in the presence of an initiator under the solution polymerization reaction conditions, wherein the alkenyl monomers comprise a monomer C' represented by formula (1) and a monomer D' represented by formula (d);

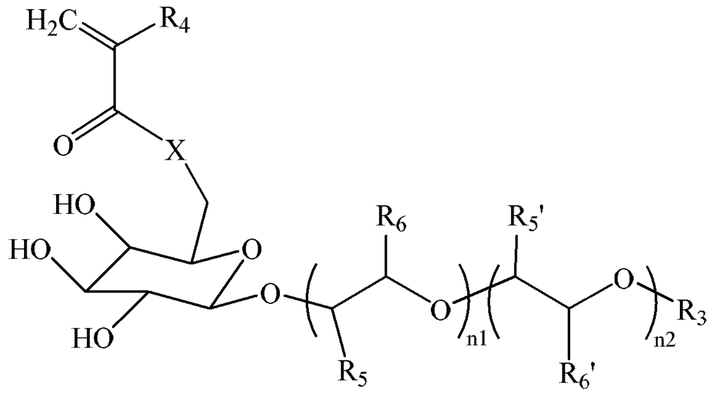

Formula (1)

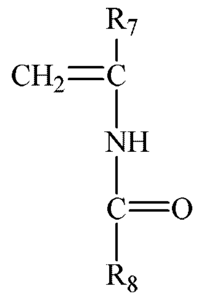

Formula (d)

Wherein $R_3$ is C4-C20 alkyl or C7-C20 alkylphenyl, $R_4$, $R_5$, $R_6$, $R_5$' and $R_6$', $R_7$ and $R_8$ are each independently H or C1-C4 alkyl, X is O or NQ, Q is H or C1-C4 alkyl, n1+ n2=0-20.

13. The method according to claim 12, wherein the weight ratio of said monomer C' to said monomer D' is (5-300): (5-300);

and/or $R_3$ is C6-C18 alkyl;
and/or $R_4$, $R_5$, $R_6$, $R_5$', $R_6$', $R_7$, $R_8$ are each independently H or methyl;
and/or when X is O, n1+ n2≠0.

14. The method according to claim 12 or 13, wherein the weight ratio of said monomer C' to said monomer D' is (6-150): (8-200);

and/or $R_3$ is n-hexyl, n-dodecyl, n-decyl, n-octyl, n-tetradecyl, 2-ethylhexyl or 11-methyldodecyl; and/or $R_5$' and $R_6$' are each independently H or methyl and $R_5$' and $R_6$' are different, both $R_5$ and $R_6$ are H;
and/or nl+n2≠0; preferably, n1 and n2 are each independently 1, 2, 3, 4, 5, 6, 7, 8, 9 or 10.

15. The method according to any one of claims 12-14, wherein the alkenyl monomers further comprise a monomer represented by formula (3-1):

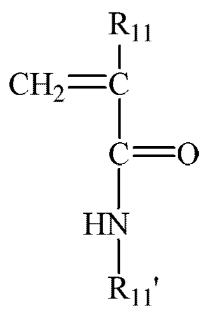

Formula (3-1)

wherein $R_{11}$ is H or C1-C4 alkyl; $R_{11}$' is H, C1-C4 alkyl, $SO_3M$-substituted C2-C6 alkyl, $P(O)(OM)_2$-substituted C2-C6 alkyl, M is H or an alkali metal element;
the weight ratio of said monomer represented by formula (3-1) to said monomer C' is (1-1.8): (0.005-0.3), preferably (1-1.7): (0.006-0.15).

**16.** The method according to any one of claims 12-15, wherein the alkenyl monomers include a monomer A' represented by formula (a), a monomer B' represented by formula (b-1) and/or (b-2), a monomer C' represented by formula (1), and a monomer D' represented by formula (D);

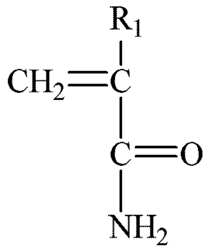 Formula (a), 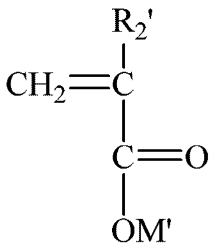 Formula (b-l),

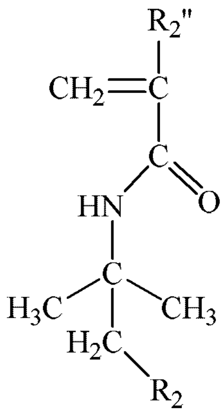 Formula (b-2), 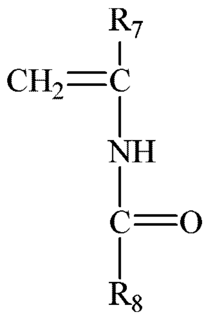 Formula (d);

wherein $R_1$, $R_2$', $R_2$" are each independently H or C1-C4 alkyl, $R_2$ is $SO_3M$ or $P(O)(OM)_2$, M and M' are each independently H or an alkali metal element;
preferably, the weight ratio of said monomer A', said monomer B', said monomer C' and said monomer D' is 1: (0.02-0.8): (0.005-0.3): (0.005-0.3), more preferably 1: (0.03-0.7): (0.006-0.15): (0.008-0.2).

**17.** The method according to any one of claims 12-16, wherein the solution polymerization reaction conditions comprise: a starting temperature of the polymerization reaction is within the range of -10°C to 30°C, a time of 2-12 hours, and a pH of 4-8.

**18.** The polymer produced with the method according to any one of claims 12-17.

**19.** A method for emulsifying and reducing viscosity of heavy oil, is **characterized in that** the method comprises: contacting a solution of the polymer according to any one of claims 4-11 and 18 with said heavy oil.

**20.** The method according to claim 19, wherein the polymer content in a solution of said polymer is within the range of 1,000-3,000 mg/L;
and/or the polymer is used in an amount of 20-50mg relative to 70g of heavy oil.

**21.** Use of the compound according to any one of claims 1-3 or the polymer according to any one of claims 4-11 and 18 in chemical flooding.

## INTERNATIONAL SEARCH REPORT

International application No.
**PCT/CN2024/122296**

**A. CLASSIFICATION OF SUBJECT MATTER**

C07H 15/08(2006.01)i; C08F212/32(2006.01)i; C08F220/56(2006.01)i; C08F220/58(2006.01)i; C08F226/10(2006.01)i; C09K8/588(2006.01)i

According to International Patent Classification (IPC) or to both national classification and IPC

**B. FIELDS SEARCHED**

Minimum documentation searched (classification system followed by classification symbols)

IPC:C07H,C08F,C09K

Documentation searched other than minimum documentation to the extent that such documents are included in the fields searched

Electronic data base consulted during the international search (name of data base and, where practicable, search terms used)

CNTXT, ENTXTC, CNKI, CAPLUS(STN), REGISTRY(STN): 丙烯酸, 丙烯酰, 丙烯酰胺, 采油, 驱油, 乳化, 聚合物, 聚氧乙烯醚, 驱油剂, 石油, 水驱开采, 糖, 糖苷, Acrylic acid, Acryloyl, Acrylamide, Oil recovery, Oil flooding, Emulsifying, Polymers, Polyoxyethylene ether, Flooding agent, Petroleum, Water Flooding, Sugar, Glycoside, 吴嘉蕾, 杨金彪, 伊卓, 张瑞琪, 赵若彤, 范荣, 胡晓娜, 李雅婧, 刘希, 尚丹森, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | WO 2014088555 A1 (EMPIRE TECHNOLOGY DEVELOPMENT LLC) 12 June 2014 (2014-06-12)<br>embodiments 2A and 3B, and claims 1-27 | 1-21 |
| A | CN 116554395 A (KOPPER CHEMICAL INDUSTRY CORP., LTD.) 08 August 2023 (2023-08-08)<br>description, paragraphs 0005-0031, and embodiment 1 | 1-21 |
| A | CN 109957384 A (CHINA PETROLEUM & CHEMICAL CORP. et al.) 02 July 2019 (2019-07-02)<br>description, paragraphs 0007-0021, and embodiment 1 | 1-21 |
| A | CN 102618239 A (CHINA NATIONAL PETROLEUM CORP.) 01 August 2012 (2012-08-01)<br>description, paragraphs 0031-0053 | 1-21 |
| A | CN 108102628 A (CHINA NATIONAL PETROLEUM CORP.) 01 June 2018 (2018-06-01)<br>description, paragraphs 0006-0025, and embodiment 1 | 1-21 |

☐ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **25 December 2024** | **02 January 2025** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/122296**

| Patent document cited in search report | Publication date (day/month/year) | Patent family member(s) | Publication date (day/month/year) |
| --- | --- | --- | --- |
| WO 2014088555 A1 | 12 June 2014 | US 2014155563 A1 | 05 June 2014 |
| | | US 9212245 B2 | 15 December 2015 |
| CN 116554395 A | 08 August 2023 | None | |
| CN 109957384 A | 02 July 2019 | None | |
| CN 102618239 A | 01 August 2012 | None | |
| CN 108102628 A | 01 June 2018 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311522670
- CN 202311522771

### Non-patent literature cited in the description


- **HE QUAN-GUO** ; **SUN YUAN-XI** ; **LIU PING et al.** Improved synthesis of AMPP based on Ritter reaction. *INDUSTRIAL WATER TREATMENT*, 2001, vol. 21, 26-28
- **SUN GANG-JIAN** ; **YANG XIU-QUAN** ; **YANG QING-LI et al.** Research on Preparation of the Glycosylated Modified Alcohol Ether and Properties thereof. *DETERGENT & COSMETICS*, 2007, vol. 37, 271-274
- *China National Standard GB/T12005.10-92*